(19) Europäisches Patentamt
European Patent Office
Office européen des brevets

(11) Publication number: **0 439 997 B1**

(12) # EUROPEAN PATENT SPECIFICATION

(45) Date of publication of patent specification: **08.11.95**

(51) Int. Cl.[6]: **C12N 15/80**, C12N 15/54, C12N 15/20

(21) Application number: **90811008.3**

(22) Date of filing: **20.12.90**

(54) **Novel fungal expression system.**

(30) Priority: **29.01.90 EP 90810068**

(43) Date of publication of application:
**07.08.91 Bulletin 91/32**

(45) Publication of the grant of the patent:
**08.11.95 Bulletin 95/45**

(84) Designated Contracting States:
**AT BE CH DE DK ES FR GB GR IT LI LU NL SE**

(56) References cited:
**EP-A- 0 120 551**
**EP-A- 0 184 438**
**EP-A- 0 225 078**
**EP-A- 0 329 203**

**CURRENT GENETICS vol. 13, 1988, pages 315-321, DE; L. DE GRAAFF et al.:**

**PROC. 4TH EUROPEAN CONGRESS ON BIOTECHNOLOGY vol. 1, 1987, page 472, Amsterdam, NL; L.H. DE GRAAFF et al.: "Structure and Expression of the Pyruvate kinase Gene of Aspergillus Nidulans: A novel selection marker in fungal transformation"**

**CURRENT GENETICS vol. 14, 1988, pages 553-560, DE; L. DE GRAAFF et al.: "Structure of the Aspergillus nidulans pyruvate kinase gene"**

(73) Proprietor: **CIBA-GEIGY AG**
**Klybeckstrasse 141**
**CH-4002 Basel (CH)**

(72) Inventor: **de Graaff, Leendert Hendrik**
**Dullertstraat 10**
**NL-6828 HK Arnhem (NL)**
Inventor: **van den Broeck, Henriette Catharina**
**Torbeckestraat 246**
**NL-6702 CA Wageningen (NL)**
Inventor: **Visser, Jacob, Prof.**
**Hinkeloordseweg 4**
**NL-6703 CK Wageningen (NL)**
Inventor: **Buxton, Frank, Dr.**
**Holderstüdeliweg 32**
**CH-4132 Muttenz (CH)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

Field of the invention

The invention relates to the field of genetic engineering and provides novel DNA molecules comprising a fungal promoter. The novel DNA molecules are useful for the construction of hybrid vectors expressing genes in filamentous fungi.

Background of the invention

Although in genetic engineering, numerous polypeptide expression systems for prokaryotic and eukaryotic hosts are already known, there is a continuing need for novel systems which have advantages over the known systems.

Very widely used as hosts are the prokaryotic Escherichia coli and the eukaryotic yeast, e.g. Saccharomyces cerevisiae, for which a large number of different expression vectors, mostly plasmids, have been developed. The drawback of E. coli hosts is that they cannot glycosylate the polypeptide. Yeasts do glycosylate, however, like E. coli they often do not secrete the polypeptides into the nutrient medium, but secrete them only into the periplasmic space. Higher eukaryotic hosts, such as mammalian cancer cells, are able to glycosylate and secrete into the nutrient medium, however, cultivation thereof is very slow and expensive and the danger exists that oncogenic nucleic acids could be isolated together with the desired peptide.

In the search for other hosts, filamentous fungi, such as Neurospora crassa, Aspergillus nidulans and Aspergillus niger, have been investigated. The application thereof in genetic engineering has lagged behind, mainly for lack of an appropriate transformation system. In contrast to Saccharomyces cerevisiae, filamentous fungi do not naturally contain plasmids which could be used for the introduction of foreign genes. It is, however, possible to transform filamentous fungi with foreign plasmids containing a selectable marker. Almost all vectors described so far for filamentous fungi do not autonomously replicate, as most of those of yeasts do, but are integrated into the fungal chromosome. Although this event occurs only at a low frequency, advantageously, integrative transformation renders the transformants mitotically very stable, even under non-selective conditions. Stable integration of more than one hundred copies has been reported.

The first vector for filamentous fungi described contained the qa-2 gene of Neurospora crassa as selectable marker [Case, M.E., Schweizer, M., Kushner, S.R. and Giles, N.H. (1979) Proc. Natl. Acad. Sci. USA 76, 5259-5263; Case, M.E. (1982) in: Genetic Engineering of Microorganisms for Chemicals (Hollander, A., DeMoss, D., Kaplan, S., Konisky, J., Savage, D. and Wolfe, R.S., eds), pp. 87-100, Plenum].

In Aspergillus nidulans, which has a sexual cycle and is therefore amenable to classical genetic manipulations, both negative and positive selection systems based on auxotrophic markers or dominant selection markers have been identified. (Ballance et al., BBRC 112, 284, 1983; Tilburn et al. Gene 26, 205, 1983; Yelton et al., PNAS 81, 1470, 984; Yelton and Timberlake, J. Cell. Biochem. Suppl. 9C, 173, 1985; Johnstone et al., EMBO J. 4, 1307, 1983; Tilburn et al., Gene 26, 205, 1983; Wernars et al., Curr. Genet. 9, 361, 1985; Kelly, J.M. et al., EMBO J. 4, 475, 1985).

Compared to N. crassa or A. nidulans, A. niger is by far the more important organism, as it is used more widely in the industrial production of enzymes, e.g. for use in the food industry. A. niger secretes a variety of hydrolytic enzymes, e.g. glucoamylase, α-amylase, pectinase, cellulase, β-glucanase, β-galactosidase, naringinase, pentosanase, acid protease and ligninase, the glucoamylase and pectinase complex being the most important ones.

Classical mutation and selection procedures in A. niger have achieved extensive strain improvements in the secretion of hydrolytic enzymes. A. niger has no known sexual cycle. Mutations can only be combined via meiotic recombination in selected diploids followed haploidination.

The heterologous amds gene (Kelly and Hynes, EMBO J. 4, 475, 1985), and the argB gene (Buxton et al., Gene 37, 207, 1985; EP 184 438; WO 86/06097), both obtained from A. nidulans, or the homologous pyrA gene (van Hartingsveldt et al., Mol. Gen. Genet. 206, 71-75, 1987; Goosen et al., Curr. Genet. 11, 499-503, 1987) have been used as selectable marker for A. niger transformation.

A. niger is the most important organism for the industrial production of pectin degrading enzymes, e.g. polygalacturonases, pectin lyases or pectin esterases.

The applications of pectin lyase, pectin esterase, polygalacturonase and enzyme mixtures thereof in fruit and vegetable processing (Table 1) have developed from the original use of pectic enzymes for treatment of soft fruit to ensure high yields of juice and pigments upon pressing and for the clarification of raw press juices. Technical enzyme preparations in use for these processes contain pectin esterases,

polygalacturonases and pectin lyases in varying amounts along with other enzymes such as arabinanases, galactanases, xylanases, cellulases, β-1,4-glucanases, glycosidases and proteases.

Table 1 (from Voragen, A.G.J., Food enzymes: prospects and limitations. In: J.P. Roozen et al., eds., Food Science: Basic Research for Technological Progress. PUDOC Wageningen, The Netherlands, 1989): Use of polygalacturonases and mixtures thereof in fruit and vegetable industry.

| Enzymes | Use |
| --- | --- |
| Polygalacturonase | Maceration, Citrus juice stabilization/viscosity reduction |
| Pectinesterase + Polygalacturonase and/or Pectin lyase | Juice clarification, juice/ oil extraction, Citrus peel oil, citrus pulp wash |
| Pectinesterase/Polygalacturonase/Pectin lyase + (Hemi-) Cellulases | Liquefaction, clear/cloudy juices Enhance natural product extraction Valorization biomass/feed |

Through the use of pectic and cellulolytic enzymes the cell walls of fruit pulps can be degraded to the stage of almost complete liquefaction. The presence of both endo- and exo-β-1,4 glucanases (cellulases) as well as pectic enzymes is essential (ref. Renard C.M.C.G. et al., Apple Protopectin: preliminary study of enzymatic extraction. In: Roozen J.P. et al., eds., Food Science: Basic Research for Technological Progress. PUDOC, Wageningen, The Netherlands, 1989).

Polygalacturonase and enzyme mixtures thereof are also useful for liquefaction and saccharifaction of biomass, for example, for the production of fermentable polysaccharides from plant cells (Beldman, G. et al., Enzyme Micros. Technol. 6, 503-507, 1984) or for the modification of pectins (for review see Voragen A.G.J. Food enzymes: prospects and limitations. In: Roozen J.P. et al., op. cit.).

In A. niger the proteins of the pectic complex are not expressed constitutively. Under inducing conditions, i.e. in the presence of pecan or breakdown products thereof A. niger expresses the above mentioned enzymes, provided that other carbon sources, such as glucose or sucrose, are limiting.

Because of the industrial importance of A. niger and of its enzymes there is a continuing need for novel A. niger expression systems for strain improvement and/or production of homologous or heterologous gene products. Of great interest, for example, is the production of individual pectin degrading enzymes or of defined mixtures thereof.

The present invention provides novel DNA molecules comprising an advantageous promoter derived from the A. niger pyruvate kinase gene (pki) which can be used for the construction of novel vectors for the expression of homologous or heterologous structural genes in filamentous fungi, especially in A. niger.

The gene encoding the A. niger pyruvate kinase (systematic name ATP:pyruvate phosphotransferase, EC 2.7.1.40) is highly expressed, indicating that transcription is under the control of a strong promoter.

A 5 kb BglII/HindIII restriction fragment of the genome of A. niger N 400 which hybridizes with a fragment from the coding region of the yeast pyruvate kinase gene was cloned. The resulting clone was named pGW1100. Cotransformation experiments with the plasmid pGW613 comprising the pyrA gene as selection marker and with pGW1100 led to increased levels of pyruvate kinase in eight out of eleven transformants investigated. From these results it was concluded that pGW1100 encodes the A. niger

pyruvate kinase and that it comprises the complete functional gene (L.H. de Graaff, The structure and expression of the pyruvase kinase gene of Aspergillus nidulans and Aspergillus niger. Ph.D. Thesis, Agricultural University of Wageningen, 1989).

Starting from this prior art novel DNA molecules comprising the pki promoter were developed in the present invention and were used for the construction of novel expression vectors for the overproduction of a homologous gene product, e.g. pectin lyase, or for the expression of a heterologous structural gene in A. niger, e.g. an interferon gene.

## Object of the invention

Objects of the invention are novel DNA molecules comprising the A. niger pki promoter, hybrid vectors useful for the expression of structural genes under the control of said promoter, hosts transformed with the novel hybrid vectors, and processes for the production of the novel DNA molecules, hybrid vectors, and transformed hosts and for the production of recombinant polypeptides by means of said transformed hosts.

## DNA molecules comprising the A. niger pyruvate kinase promoter

The present invention concerns the A. niger pyruvate kinase (pki) promoter which is comprised in the DNA molecule which has the nucleotide sequence with the sequence identification No. (SEQ ID NO.) 1, or a derivative or a fragment thereof with promoter activity.

The nucleotide sequence with the SEQ ID NO. 1 comprises the whole functional pyruvate kinase gene pki of A. niger including the promoter. The pki promoter extends from nucleotide position 1 up to the first nucleotide of the coding region for pyruvate kinase in position 1042. The pki promoter binds to RNA polymerase as well as to regulatory proteins and can control the expression of a structural gene operatively linked therewith. The A. niger pki promoter is a strong promoter. The invention concerns accordingly a DNA molecule of the nucleotide sequence which extends from about position 1 up to about 1042 or a fragment or derivative of this promoter retaining a promoter activity which may be regulated or not.

The complete pki promoter of A. niger can be regulated because it comprises regulatory elements which render the level of transcription dependent on the carbon source used for cultivating A. niger cells comprising said promoter. Use of a gluconeogenic carbon source such as acetate leads to a low level of transcription whereas the use of a glycolytic carbon source, e.g. glucose, leads to a high level of transcription.

In the promoter comprised in nucleotide sequence with SEQ ID NO. 1 a potential TATA box is located between nucleotide positions 927 and 934, a potential CAAT box between positions 830 and 836 and extensive CT rich regions (CT blocks) between positions 848 and 1041. The latter can be found in promoter/regulation regions of many highly expressed genes originating from yeasts and fungi.

A fragment of the invention is, for example, a fragment selected from the group of fragments with promoter activity which start with any of the nucleotides in position 1 up to about position 950 and end with a nucleotide in about position 1041 in the nucleotide sequence with the SEQ ID NO 1. Preferred is a fragment starting with a nucleotide in about position 300 and ending with a nucleotide in about position 1041 in said nucleotide sequence. The fragments of the invention may, for example, start at a restriction enzyme cleavage site located within the nucleotide sequence with the SEQ ID NO. 1, e.g. the BamHI site (in about position 300) or the following sites (approximate positions in brackets): SphI (441), SmaI (859), XmaI (859).

A most preferred fragment is the fragment which starts at the BamHI site in about position 300 and extends up to position 1041. This fragment is part of the BamHI/PvuII fragment which extends from the BamHI site in about position 300 up to the PuvII site in about position 1352 and which is used hereinafter in the examples for inserting a restriction enzyme cleavage site.

A derivative according to the invention is, for example, a mutant, a recombinant derivative, or a recombinant derivative of a mutant of the pki promoter comprised in the DNA molecule having the nucleotide sequence with the SEQ ID NO. 1 or of a fragment thereof. A derivative according to the invention comprises a sequence with pki promoter activity which can be regulated or not.

A mutant according to the invention may be a naturally occuring or preferentially an artificial mutant. Mutants of the pki promoter of the invention are in particular such having new restriction enzyme cleavage sites, for example for the restriction enzymes NsiI, BamHI, EcoRI, HindIII, PstI, SalI, NcoI and the like. Preferred is a mutant comprising a new restriction enzyme cleavage site in or about position 1041 of the sequence with SEQ ID NO. 1, which can be used for inserting a structural gene 3' of the promoter which then is operatively linked therewith. Such a mutant is, for example, characterized in that the nucleotide

sequence with the SEQ ID NO. 2 substitutes the sequence extending between position 1034 and 1054 in the nucleotide sequence with SEQ ID NO. 1 and in that a NsiI site is located immediately adjacent to position 1041 of SEQ ID NO 1.

A mutant according to the invention is also a mutant of a fragment the meaning of which is given hereinbefore. A preferred mutant of a fragment comprises downstream of the pki promoter a new restriction enzyme cleavage site for operatively linking a structural gene to the promoter. Such a preferred mutant of a fragment is, for example, comprised in the 1053 bp BamHI/PvuII fragment or the 742 bp BamHI/NsiI fragment comprised in the M13mp18-PK (BamHI-NsiI-PvuII) RF DNA. The 1053 bp BamHI/PvuII fragment of M13mp18-PK (BamHI-NsiI-PvuII) has a nucleotide sequence extending from the BamHI site in about position 300 up to the PvuII site in about position 1352 of a sequence with the SEQ ID NO. 1 which is mutated in that the sequence with SEQ ID NO. 2 substitutes the nucleotides from position 1034 to 1054. The 742 bp BamHI/NsiI fragment extends from said BamHI site up to the NsiI site in the substituted region. In both mutants, a NsiI site is located immediately adjacent to position 1041 of SEQ ID NO 1.

A derivative according to the invention is also a recombinant DNA molecule. Such a recombinant DNA molecule is characterized, for example, in that it contains an oligonucleotide linker attached at a 3' and/or 5' end of a DNA molecule of the invention with pki promoter activity which provides for successful linkage to other DNA molecules, e.g. vector sequences. The linker may comprise one or more restriction enzyme cleavage sites and/or partially single stranded ends ("sticky ends") and/or blunt ends.

A derivative according to the invention is also a recombinant DNA molecule comprising a DNA molecule of the invention with pki promoter activity and a homologous or heterologous structural gene with or without introns, except the homologous pki structural gene, which is operatively linked with the pki promoter, and optionally an oligonucleotide linker. A derivative according to the invention may comprise a further expression control sequence in addition to the pki promoter which is also operatively linked with said structural gene. Such an expression control sequence is, for example, an enhancer, a transcriptional terminator, a ribosomal binding region, a translational initiation or termination site, or a signal sequence , e.g. a signal sequence of an A. niger pectin lyase gene, e.g. for PLA, B, C or D, or polygalacturonase gene, e.g. for PGI, PGC or PGII.

Homologous structural genes are those which are derived from A. niger. They are coding, for example, for enzymes, preferentially such as are useful in industry, e.g. food and feed industry. Such enzymes are, for example, pectinolytic enzymes such as pectin esterase, endo- and exo-acting polygalacturonase (PG), pectin lyase (PL), rhamnogalacturonase or arabinases, galactanases, xylanases, cellulases, $\beta$-1,4-glucanases, glycosidases, and the like.

The nucleotide sequence of the structural gene for the A. niger PLD is disclosed in the European patent application with the publication No. EP-A2-0 278 355. The structural genes for further A. niger PLs, particularly PLA, B, C, E and F, are disclosed in the European patent application with the publication No. EP-A2-0 353 188. The structural genes for A. niger PGs, particularly PGII, are disclosed in the GB patent application with the application No. 8919884.0.

E. coli HB101 or JM109 cells transformed with plasmids pGW 820, 830, 850 and 1800 comprising A. niger structural genes, are deposited according to the Budapest Treaty at the Deutsche Sammlung für Mikroorganismen und Zellkulturen, Mascheroder Weg 1b, D-3300 Braunschweig. Nucleotide sequences of the parts of the structural gene for PGII which encode the N- and C-terminal, respectively, are depicted in the sequence listing. For further information see Table 2.

Table 2

| Structural gene for | Transformed E. coli | Deposition No. | SEQ ID NO. |
|---|---|---|---|
| PLA | HB101 / pGW820 | DSM 4388 | |
| PLB | HB101 / pGW830 | DSM 4389 | |
| PLC | HB101 / pGW850 | DSM 4390 | |
| PGII | JM109 / pGW1800 | DSM 5505 | 3 (N-terminal)<br>4 (C-terminal) |

Homologous genes within the scope of the invention are also derivatives of the PL and PG genes mentioned hereinbefore, including fragments, mutants and DNA sequences which are generated in accordance with the genetic code in that an unlimited number of nucleotides are replaced by other nucleotides without changing the amino acid sequence of the encoded polypeptide.

Heterologous structural genes originate from viruses, procaryotic cells or eucaryotic cells and may be derived from genomic DNA or from cDNA prepared via the mRNA route or may be synthesized chemically, and are coding for a wide variety of useful polypeptides, including glycosylated polypeptides, in particular of higher eukaryotic, especially mammalian, such as animal or especially human origin, such as enzymes which can be used, for example, for the production of nutrients and for performing enzymatic reactions in chemistry, or polypeptides which are useful and valuable for the treatment of human or animal diseases or for the prevention thereof, for example, hormones, polypeptides with immunomodulatory, anti-viral and antitumor properties, antibodies, viral antigens, vaccines, clotting factors, foodstuffs and the like.

Examples of such structural genes are e.g. those coding for hormones such as secretin, thymosin, relaxin, calcitonin, luteinizing hormone, parathyroid hormone, adrenocorticotropin, melanoycte-stimulating hormone, $\beta$-lipotropin, urogastrone or insulin, growth factors, such as epidermal growth factor, insulin-like growth factor (IGF), e.g. IGF-I and IGF-II, mast cell growth factor, nerve growth factor, glia derived nerve cell growth factor, or transforming growth factor (TGF), such as TGF$\alpha$ or TGF$\beta$, e.g. TGF$\beta$1, $\beta$2 or $\beta$3, growth hormone, such as human or bovine growth hormones, interleukin, such as interleukin-1 or -2, human macrophage migration inhibitory factor (MIF), interferons, such as human $\alpha$-interferon, for example interferon-$\alpha$A, $\alpha$B, $\alpha$D or $\alpha$F, $\beta$-interferon, $\gamma$-interferon or a hybrid interferon, for example an $\alpha$A-$\alpha$D- or an $\alpha$B-$\alpha$D-hybrid interferon, especially the hybrid interferon BDBB, proteinase inhibitors such as $\alpha_1$-antitrypsin, SLPI and the like, hepatitis virus antigens, such as hepatitis B virus surface or core antigen or hepatitis A virus antigen, or hepatitis nonA-nonB antigen, plasminogen activators, such as tissue plasminogen activator or urokinase, tumour necrosis factor, somatostatin, renin, $\beta$-endorphin, immunoglobulins, such as the light and/or heavy chains of immunoglobulin D, E or G, or human-mouse hybrid immunoglobulins, immunoglobulin binding factors, such as immunoglobulin E binding factor, e.g. sCD23, calcitonin, human calcitonin-related peptide, blood clotting factors, such as factor IX or VIIIc, erythropoietin, eglin, such as eglin C, hirudin, desulfatohirudin, such as desulfatohirudin variant HV1, HV2 or PA, human superoxide dismutase, viral thymidin kinase, $\beta$-lactamase, glucose isomerase. Preferred genes are those coding for a human $\alpha$-interferon or hybrid interferon, particularly hybrid interferon BDBB, human tissue plasminogen activator (t-PA), hepatitis B virus surface antigen (HBVsAg), insulin-like growth factor I and II, eglin C and desulfatohirudin, e.g. variant HV1. In a DNA molecule of the present invention, the present promoter is operably linked to the polypeptide coding region so as to ensure effective expression of the polypeptide.

The DNA molecules of the invention, including fragments and derivatives thereof, can be used for screening DNA gene libraries or mRNA for further similar DNAs or mRNAs.

The invention concerns further hybrid vectors comprising as insert a DNA molecule of the invention comprising the pki promoter activity. Said DNA molecules of the invention include the pki promoter, fragments or derivatives thereof, e.g. mutants or the fusion of a structural gene mentioned hereinbefore with the promoter or a fragment thereof. The hybrid vectors of the invention are usable for cloning in hosts, such as bacteria, fungi or animal cells. Such hybrid vectors are derived from any vector useful in the art of genetic engineering, such as from phages, cosmids, plasmids or chromosomal DNA, such as derivatives of phage $\lambda$, e.g. NM 989, or of phage M13, e.g. M13mp18 or M13mp19 phage DNA, bacterial plasmids, e.g. pBR322, pUN121, pUC18, or yeast plasmids, e.g. yeast 2$\mu$ plasmid, or also chromosomal DNA, derived e.g. from Aspergillus, e.g. A. niger, for example those provided by EP 184 438, or defective phages or defective plasmids in the presence of a helper phage or a helper plasmid allowing replication of said defective phages or plasmids, e.g. M13(+)KS vector in presence of e.g. M13K07 helper phage.

A hybrid vector of the invention comprises, in addition to the DNA molecules of the invention, a replication site and, if required, a marker gene and/or an additional expression control sequence other than the pki promoter, e.g. an enhancer, a transcriptional terminator, a ribosomal binding region, a translational initiation or termination site, or a signal sequence, e.g. the signal sequence of the structural gene for A. niger pectin lyase A, B, C or D, or polygalacturonase, e.g. PGI or II.

A hybrid vector of the invention is not pGW1100.

However, a hybrid vector of the invention may comprise fragments of pGW1100 with pki promoter activity. An example of such a hybrid vector is M13mp18-PK (BamHI-PvuII), which comprises the 1053 bp BamHI/PvuII restriction fragment extending from the BamHI site in about nucleotide position 300 up to the PvuII site in about nucleotide position 1352 of the sequence with SEQ ID NO. 1.

Another hybrid vector of the invention is such comprising a mutation in the A. niger pki promoter. Said mutation may generate a new restriction enzyme cleavage site, particularly such which is suitable for the insertion of a structural gene which then is operatively linked with the pki promoter. An example of such a vector is in particular the M13mp18-PK (BamHI-NsiI-PvuII) which comprises a mutated DNA molecule of the invention with a new NsiI site at the translation initiation site of the A. niger pyruvate kinase structural gene.

A hybrid vector of the invention may also comprise a homologous structural gene except the A. niger pyruvate kinase structural gene, or a heterologous structural gene which is operatively linked with the pki promoter. Such a hybrid vector is, for example, pPK-PLB which comprises the 742 bp BamHI/NsiI fragment of M13mp18-PK (BamHI-NsiI-PvuII) and the 2.6 kb BamHI/NsiI fragment of pGW830. Said hybrid vector comprises the structural gene encoding PLB including its signal sequence.

Preferred hybrid vectors are pPKI-IFN-2, pPKIssIFN-2, pPK-PLB, M13mp18-PK (BamHI-PvuII), M13mp18-PK (BamHI-NsiI-PvuII).

Process for the Preparation of the DNA molecules comprising the A. niger pki promoter, of hybrid vectors comprising such a DNA molecule and hosts transformed with said hybrid vectors

Further object of the invention is a process for the preparation of a DNA molecule of the invention, e.g. a process comprising preparing such a DNA molecule by an in vitro synthesis or culturing a host which comprises such a DNA sequence.

The culturing of hosts is carried out in a conventional nutrient medium which may be supplemented with or deprived of chemical compounds allowing negative or positive selection of the transformants, i.e. such hosts containing the desired DNA molecule together with a selection marker, from the non-transformants, i.e. such hosts lacking the desired DNA molecule.

Any transformable host useful in the art may be used, e.g. bacteria, such as E. coli, fungi, such as Saccharomyces cerevisiae, or in particular filamentous fungi, such as Aspergillus, e.g. A. nidulans, A. oryzae, A. carbonarius, A. awamori, A. japonicus and especially A. niger. Transformation of the hosts is carried out by conventional methods.

A DNA molecule of the invention can be obtained from Aspergillus niger containing the pyruvate kinase gene (pki), in particular from a genomic library thereof or also via a mRNA used to prepare a cDNA molecule.

In the following the preparation of a DNA molecule of the present invention is described in more detail.

A genomic library can be prepared e.g. by partial digestion of genomic DNA of an A. niger strain, e.g. NW756 or N400, with e.g. Sau3AI or MboI, and cloning the high molecular weight DNA fragments in a suitable host vector, e.g. the E. coli plasmid pUN121 or a lambda vector, e.g. EMBL4.

In order to successfully screen the genomic library for DNA sequences comprising the pki, a hybridizing DNA probe is necessary. This can be a synthetic DNA probe, or another pyruvate kinase gene or a part thereof, which hybridizes to the A. niger pki, for example the yeast pyruvate kinase structural gene comprised in the plasmid pPYK1 (Burke et al., 1983).

For screening purposes the DNA probes are radioactively labelled by methods known in the art, e.g. at the 5' end using $\gamma^{32}$P-ATP and T4 kinase. Host microorganisms carrying nucleic acids of the present invention as an insert are identified by hybridization with the labelled DNA probe on filter replicas of the gene library. The DNA molecules hybridizing with the probe are isolated and, if desired, are subcloned according to conventional methods. The plasmid pGW1100 is such a subclone of a genomic clone from an A. niger N400 library. It comprises a 5.0 kbp BglII/HindIII fragment of the A. niger genome which comprises the entire functional gene for the A. niger pyruvate kinase.

The identified pki or gene fragments are then sequenced. The full sequence of the functional pki of A. niger comprised in pGW1100 is depicted in the sequence listing under SEQ ID NO. 1.

The plasmid pGW1100 is used to prepare DNA molecules of the invention. Such DNA molecules are prepared in conventional manner by applying conventional restriction enzymes, linkers, mutation, ligation, amplification and isolation processes.

Fragments of a DNA molecule with the nucleotide sequence with the SEQ ID NO. 1 are, for example, prepared by a method comprising treating said DNA molecule with nucleases, e.g. exonucleases such as Bal31 or ExoIII, or endonucleases such as restriction enzymes.

Derivatives of a DNA molecule having the nucleotide sequence with the SEQ ID NO. 1 or of a fragment thereof are prepared, for example, by mutagenesis according to conventional methods [see review article of M.J. Zoller and M. Smith, Methods Enzymol. 100, 468 (1983), D. Botstein and D. Shortle, Science 229, 1193 (1985) or K. Norris et al., Nucl. Acids Res. 11, 5103 (1983)], for example as described in Example 2.1.2. hereinafter.

A mutant containing a new restriction site can be prepared, for example, by site-directed mutagenesis using a mutagenic oligonucleotide according to conventional methods. An example for such a mutagenic oligonucleotide for introducing a mutation into the sequence with SEQ ID NO. 1 is the DNA molecule depicted under SEQ ID NO. 2.

A recombinant derivative of a DNA molecule having the nucleotide sequence with the SEQ ID NO. 1, of a fragment or a mutant thereof can be prepared, for example, by recombinant DNA technology, e.g. by a method comprising cutting such a DNA molecule, fragment or mutant thereof with a restriction enzyme and/or ligating it with another DNA molecule, e.g. with an oligonucleotide linker or with a DNA molecule comprising a structural gene, signal sequence and/or terminator region.

All DNA molecules of the present invention can also be prepared by an in vitro synthesis according to conventional methods. The in vitro synthesis is especially applicable for the preparation of smaller DNA molecules of the invention.

A hybrid vector of the invention is prepared according to conventional methods of genetic engineering, e.g. by a method comprising ligating a vector useful in the art of genetic engineering which is linearized, e.g. by cutting with a restriction enzyme, with a DNA molecule of the invention, transforming a suitable host cell with the ligation mixture, and identifying and/or selecting transformants.

Host cells are transformed by a conventional method and the transformants are identified and/or selected, for example, by their resistance, e.g. against tetracycline, or by complementation of auxotrophic markers, e.g. pyrA.

In particular the described expression vectors are amplified in suitable E. coli host strains, such as HB101, JM109, MH1, DH5α and the like, transformed and selected by methods conventional in the art. The amplified plasmid DNA is isolated from the bacteria by conventional methods, in particular as described by Birnboim & Doly (Nucleic Acids Res. 7, 1513-1523, 1979).

A DNA molecule of the invention or a hybrid vector of the invention, in particular such comprising a homologous or heterologous structural gene, can also be used to transform filamentous fungi, such as Aspergillus, Trichoderma, Penicillium or Cephalosporium, e.g. A. nidulans, A. japonicus, A. oryzae, A. carbonarius, A. awamori and especially A. niger.

In order to allow selection of the transformed from the nontransformed fungi, the hybrid vectors of the invention may carry a selection marker or, alternatively, the fungi may be cotransformed with a second vector containing such a selection marker. As in other systems such selection marker is an expressible structural gene, the expressed polypeptide of which provides resistance against compounds toxic to the recipient or which completes the enzyme system of a mutant lacking such essential polypeptide. Such marker genes are for example the known qa-2, pyrG, pyr4, trpC, amdS or argB genes.

As described in EP 278.355 a marker gene, named pyrA, was isolated from the genomic library of A. niger, which is related to and has similar function as pyrG of A. nidulans and pyr4 of N. crassa, namely producing the enzyme orotidine 5'-phosphate decarboxylase. This enzyme catalyses the decarboxylation of orotidine 5'-phosphate to uridylic acid (uridine 5'-phosphate) and also of fluoro-orotic acid to the toxic fluoro-uridine. From E. coli Bg5183/pCG59D7 which is deposited at the Deutsche Sammlung von Mikroorganismen under DSM 3968, the plasmid pCG59D7 comprising the pyrA gene was isolated and used for cotransformation of an A. niger $pyrA^-$ mutant. Such $pyrA^-$ mutant is defective in the orotidine 5'-phosphate decarboxylase gene and therefore is unable to produce the corresponding enzyme. Such mutants are, for example, A. niger N593 or A. niger An8, which latter is deposited at the Deutsche Sammlung von Mikroorganismen under No. DSM 3917. The mutants are prepared by treating conidiospores of A. niger under mutating UV-irradiation and colonies surviving in the presence of fluoro-orotic acid and uridine are selected. Colonies surviving in the presence of fluoroorotic acid and absence of uridine are eliminated.

The invention concerns further hosts transformed with a hybrid vector of the invention. Such transformants are for example bacteria, such as E. coli, or filamentous fungi, such as Aspergillus, Penicillium or Cephalosporium, and in particular A. nidulans, A. japonicus, A. oryzae, A. carbonarius A. awamori or preferably A. niger, e.g. A. niger An8 or N593.

In particular preferred is E. coli JM101 or DH5α transformed with pPKI-IFN-2, pPKIssIFN-2 or pPK-PLB, Aspergillus niger N593 or An8 transformed with pPK-PLB, pPKI-IFN-2 or pPKIssIFN-2 and optionally with the selection marker plasmid pGW613 or pCG59D7.

The invention concerns also a method for the preparation of such transformants comprising treatment of a host under transforming conditions with a recombinant DNA molecule or a hybrid vector of the invention, optionally together with a selection marker gene, and, if required, selecting the transformants.

Process for the preparation of polypeptides

The invention concerns further a method for the preparation of polypeptides, characterized in that a structural gene coding for a polypeptide, e.g. such as those the meaning of which is given hereinbefore, preferentially those coding for a human α-interferon or hybrid interferon, particularly hybrid interferon BDBB, human tissue plasminogen activator (t-PA), hepatitis B virus surface antigen (HBVsAg), insulin-like growth

factor I and II, eglin C and desulfatohirudin, e.g. variant HV1, is operatively linked with the pki promoter and is expressed in a suitable host. When required, the polypeptide is isolated in a conventional manner. Depending on the construction of the vector the products are either produced in the host cell or, if a signal sequence is present, are produced in the cell and secreted. A suitable host is preferentially an Aspergillus species, e.g. A. niger, in particular A. niger An8 or N593. A suitable host is also another filamentous fungus, e.g. Neurospora crassa, or a yeast, e.g. Saccharomyces cerevisiae or Kluyveromyces lactis.

It is possible to produce a single gene product, whereby various methods can be applied. For example, a method for the production of a single polygalacturonase PG or pectin lyase PL, preferentially PLB, is characterized in that a suitable host which is not capable of expressing any PG or PL or which expresses PGs or PLs in low amount, is transformed with a hybrid vector comprising a structural gene coding for a PG or PL, e.g. PLB, and that said gene is expressed. Using the pki promoter as control region it is now also possible to produce a single gene product, e.g. PLB, in a suitable transformed host which can produce the corresponding or a related endogenous gene product(s), e.g. PLA, B, C, D, E or F, only under inducing conditions, e.g. if pectin or pectin breakdown products are in the medium, by culturing said transformed host under conditions which do not allow the expression of the endogenous structural gene(s) but only the expression of the structural gene which is under the control of the pki promoter. Such a condition is given, for example, if a minimal medium with glucose as carbon- and energy source is used. If a host not capable of expressing any PG or PL is used or if a condition not allowing the production of endogenous PLs is applied, said single PG or PL can be obtained in pure form, that means uncontaminated by any other PG or PL.

The single gene product which is produced in a suitable host transformed with a DNA molecule or hybrid vector of the invention which is coding for such gene product, and physiologically acceptable salts thereof are also subjects of the present invention. Preferred are enzymes of A. niger, particularly such which are useful in food and feed industry, e.g. PGs or PLs, in particular PLB. The invention concerns said polypeptides whenever produced by a method according to the present invention.

The invention concerns further a process for the preparation of enzymatic compositions comprising one or more of a polypeptide produced by a method according to the invention, e.g. of a single PL and/or a derivative thereof with PL activity and/or of a single PG and/or a derivative thereof with PG activity and/or physiologically acceptable salts thereof optionally in a predetermined combination with one or more suitable enzymes having other than a PL or PG activity, respectively.

Suitable enzymes having other than PL activity are degrading and modifying cellular polymers. Such enzymes are e.g. pectin esterases, endo- and exo-acting polygalacturonases, cellulases, mixed endo-glucanases, hemicellulases, xylanases, arabinases, galactanases, $\alpha$- and $\beta$-glycosidases, and the like.

Suitable enzymes having other than PG activity are degrading and modifying cellular polymers. Such enzymes are e.g. pectin esterases, pectin lyases, cellulases, mixed endo-glucanases, hemicellulases, xylanases, arabinases, galactanases, $\alpha$- and $\beta$-glycosidases, and the like.

Single PGs or PLs or derivatives thereof with PG or PL activity, respectively, produced according to a process of the invention, or enzymatic compositions thereof are useful e.g. for clarification of vegetable or fruit juice, for the enhancing of the juice yield in vegetable or fruit juice production and of pressing yield of oil-containing seeds or fruits, for stabilization of vegetable or fruit juice, for reduction of the viscosity of vegetable or fruit juice, for the liquefaction of biomass, for maceration, for the enhancement of natural product extraction like natural pigments, aromas and flavours, for the valorization of biomass, food or feed, and the like.

The invention most preferentially concerns the A. niger pki promoter or a fragment or derivative thereof with promoter activity, a hybrid vector, a transformed host, a process for the preparation of the A. niger pki promoter or a fragment or derivative thereof with promoter activity, a process for the preparation of a hybrid vector, a process for the preparation of a transformed host, and a process for the preparation of a polypeptide, as herein described in the examples.

The following examples serve to illustrate the invention, however are in no way intended to restrict it.

The abbreviations have the following meanings:

| | |
|---|---|
| Amp | ampicillin |
| ATP | adenosine triphosphate |
| BSA | bovine serum albumin |
| bp | base pairs |
| CIP | calf intestine alkaline phosphatase |
| dATP | 2'-deoxy-adenosine triphosphate |

| | |
|---|---|
| dCTP | 2'-deoxy-cytidine triphosphate |
| d.e. | degree of esterification |
| dGTP | 2'-deoxy-guanosine triphosphate |
| DNA | deoxyribonucleic acid |
| dNTP | 2'-deoxy-nucleotide triphosphate |
| DTT | 1,4-dithiothreitol |
| dTTP | 2'-deoxy-thymidine triphosphate |
| EDTA | ethylenediaminetetraacetic acid disodium salt |
| EGTA | bis-(aminoethyl)-glycolether-N,N,N',N'-tetraacetic acid |
| IFN | interferon |
| kDa | kilodalton |
| kbp | kilobasepairs |
| Km | Michaelis-Menten constant |
| LMP | low melting point |
| O.D. | optical density |
| PCR | polymerase chain reaction |
| PEG | polyethyleneglycol |
| pki | pyruvate kinase gene of A. niger |
| PLB | pectin lyase B |
| RNA | ribonucleic acid |
| rpm | rotations per minute |
| SDS | sodium dodecyl sulfate |
| SSC | sodium sodium citrate (0.15 M NaCl, 0.015 M sodium citrate) |
| Tc | tetracycline |
| Tris | tris(hydroxymethyl)-aminomethane |
| U | units |

Media:

| | |
|---|---|
| LC medium | 1 % trypticase peptone (BBL), 0.5 % yeast extract (BBL), 0.8 % NaCl, 1 ml Tris-HCl pH 7.5 per litre |
| 2xYT medium | per litre 16 g trypticase peptone (BBL), 10 g yeast extract, 5 g NaCl |
| minimal medium for A. niger | 1 litre contains 1.5 g $KH_2PO_4$, 0.5 g KCl, 0.5 g $MgSO_4.7H_2O$, 4.0 g $NH_4Cl$, 10.0 g glucose, traces of $FeSO_4$, $MnSO_4$, $ZnCl_2$, adjusted to pH 6.5 with NaOH |
| complete medium for A. niger | minimal medium plus 0.2 % trypticase peptone for (BBL) 0.1 % casaminoacids (Difco), 0.1 % yeast extract (BBL), 0.05 % ribonucleic acid sodium salt from yeast (ICN, Cleveland, USA), 2 ml vitamin solution per litre. |
| vitamin solution | per 100 ml 10 mg thiamine, 100 mg riboflavin, 10 mg pathotenic acid, 2 mg biotin, 10 mg p-aminobenzoic acid, 100 mg nicotinamide, 50 mg pyridoxin-HCl |

For plates all media are solidified by addition of 1.5 % agar (BBL), for topagar(ose) 0.7 % agar (BBL) or agarose (Seakem) is used.

Following strains are used:

E. coli JM 101 (Messing, 1979)
E. coli RZ 1032 (Pharmacia)
E. coli DH5α (Bethesda Research Laboratories)
E. coli DH5αF' (Bethesda Research Laboratories)
E. coli BW313 (Kunkel, 1985)
A. niger An8 (DSM 3917, EP-A-0278355)
A. niger N593

The following vectors are used:

pBR322

Described in Sutcliffe, J.G. (1979), Peden, K.W.C. (1983) or Bolivar et al. (1977)

pGW613

This plasmid has been described by Goosen et al. (1987).

M13mp phage

The M13mp18 and M13mp19 vectors (Norrander et al., 1983) are derivatives of the single-stranded DNA bacteriophage M13 and are designated to facilitate DNA sequencing by allowing cloning of DNA fragments at a versatile polylinker site and the cloning of the same restriction fragment in both possible orientations. Sequences cloned into these vectors can readily be used as templates for sequencing reactions or the production of single-stranded probes using stranded oligodeoxyribonucleotide primer and the Klenow fragment of the E. coli DNA polymerase I. The vector DNA is carrying the E. coli lac-operon promoter and the genetic information of the first 145 amino acids of $\beta$-galactosidase. The polylinker sequences containing multiple restriction sites are inserted into the lacZ sequence. The polylinker retains the LacZ reading frame and the vector gives allelic complementation of a LacZ$\alpha$ host strain, yielding blue plaques on plates containing IPTG and X-gal. Recombinant phages containing inserts that destroy the reading frame or otherwise interfere with expression of the lacZ$\alpha$ peptide are revealed as colorless plaques.

pCG 59D7

This plasmid is described in EP 88 101 397.3 and can be obtained from Escherichia coli BJ5183/pCG59D7 (DSM 3968). The plasmid comprises the pyrA gene and is used for cotransformation of A. niger pyrA$^-$ mutants.

M13 K07

Helper M13 phage, e.g. for M13(+)KS. Described in Mead et al. (1986) and Dotto and Zinder (1984).

pPYK1

Comprises the S. cerevisiae pyruvate kinase gene. Described in Burke et al., 1983.

pGW1800

Comprises the PGII gene of A. niger. An E. coli JM109 strain comprising pGW1800 is deposited as DSM 5505 at the Deutsche Sammlung von Mikroorganismen.

pGW830

Comprises the pelB gene of A. niger. An E. coli HB101 strain comprising pGW830 is deposited as DSM 4389 at the Deutsche Sammlung von Mikroorganismen.

pTZ18R

Obtainable from Pharmacia.

pGW1100

Comprises the A. niger pki gene. Described in L.H. de Graaff, 1989 and deposited as E. coli DH5$\alpha$F'/pGW1100 under No. 5747 at the Deutsche Sammlung von Mikroorganismen.

pJDB207-IFNAM119

Described in the European patent application EP-A-0 205 404.

Example 1: Isolation and characterization of the A. niger pyruvate kinase gene

Example 1.1: Isolation of a DNA fragment comprising the yeast pyruvate kinase gene

The plasmid pPYK1 comprising the 1.8 kb EcoRI fragment from the Saccharomyces cerevisiae pyruvate kinase gene (Burke et al., 1983) is digested with EcoRI, using the conditions given by the supplier (BRL). The resulting fragments are separated by electrophoresis in a 0.6 % low melting point (LMP) agarose gel. The piece of LMP agarose containing the 1.8 kb EcoRI fragment is cut from the gel, and the DNA is extracted by the following procedure: TE buffer (10 mM Tris/HCl, 1 mM EDTA, pH 8.0) is added to the agarose piece to a final volume of 500 µl, followed by 250 µl phenol. The agarose is melted by incubation at 65 °C for 10 min and mixing of the solution. After addition of 250 µl CIA (chloroform/isoamylalcohol 24:1) the aqueous and organic phases are separated by centrifugation (Eppendorf centrifuge) for 10 min at 14,000 x g. The organic phase is discarded and the aqueous phase is extracted once again by incubating with ½ vol of phenol for 10 min at 65 °C, subsequent addition of another ½ vol of CIA and separation of the phases. The organic phase is discarded again and the aqueous phase is sequentially extracted at room temperature with an equal volume of phenol/CIA (1:1) and with an equal volume of CIA. Finally the DNA is precipitated from the aqueous phase by adding 0.1 vol 3 M NaAcetate, pH 5.6 and 2 vol ethanol. The DNA is sedimented by centrifugation (Eppendorf centrifuge) for 30 min at 14,000 x g at room temperature. After removal of the supernatant the DNA pellet is dried using a Savant Speedvac® vacuum centrifuge. The DNA is finally dissolved in 10 µl TE, and the concentration is determined by agarose electrophoresis, using a known amount of comigrating lambda DNA as reference.

Example 1.2: $^{32}$P-labelling of DNA fragments

100 ng of the 1.8 kb EcoRI fragment isolated from the plasmid pPYK1 as described in Example 1.1 is labelled by nick translation, essentially as described by Maniatis et al., 1982, pp. 109 to 112. Five µl $\alpha$-$^{32}$P-dATP (10 µCi/µl, 3000 Ci/mMol), 1 µl (5 U/µl) DNA polymerase I, 100 pg DNase I, 100 ng 1.8 kb EcoRI fragment of pPYK1 and sterile water are added to 40 µl reaction buffer (consisting of 25 µM dGTP, 25 µM dCTP, 25 µM dTTP, 5 mM $MgCl_2$, 50 mM Tris/HCl pH 7.5) to a final volume of 50 µl. The reaction mixture is incubated for two h at 16 °C. The reaction is stopped by the addition of 5 µl 500 mM EDTA, pH 8.0. To remove the unincorporated $\alpha$-$^{32}$P-dATP from the mixture, the volume is enlarged to 100 µl with TE and the $\alpha$-$^{32}$P-dATP is removed by fractionation on a Sephadex G50 (Pharmacia) column. Fractions containing the radioactively labelled 1.8 kb EcoRI fragment are collected and pooled. The labelled DNA is denatured by incubation for three min at 100 °C and kept single stranded by rapid chilling on ice, before it is added to the hybridization solution described in Example 1.4.

Example 1.3: Isolation of high molecular weight DNA from A.niger

The A. niger DNA is isolated by a slightly modified procedure used to isolate plant RNA (Slater, 1985). Mycelium which is grown overnight in liquid minimal medium is harvested, washed with cold saline, frozen in liquid nitrogen and stored at -80 °C. Nucleic acids are isolated by disrupting 0.5 g frozen mycelium using a microdismembrator (Braun). The mycelial powder obtained is extracted with freshly prepared extraction buffer. The extraction buffer is prepared as follows: 1 ml tri-isopropylnaphtalene sulfonic acid (TNS) (20 mg/ml) is thoroughly mixed with 1 ml p-aminosalicylic acid (PAS) (120 mg/ml) and 0.5 ml 5 x RNB buffer is added (5 x RNB contains 121.10 g Tris, 73.04 g NaCl and 95.10 g EGTA in 1 l, pH 8.5). After the addition of 1.5 ml phenol, the extraction buffer is equilibrated for 10 min at 55 °C. The warm buffer is then added to the mycelial powder, and the suspension is thoroughly mixed for 1 min. After addition of 1 ml chloroform the suspension is remixed for 1 min. After centrifugation at $10^4$ x g for 10 min the aqueous phase is extracted with an equal volume of phenol/chloroform (1:1) and is then extracted twice with chloroform. DNA is isolated from the aqueous phase using the following procedure: The DNA is immediately precipitated from the aqueous phase with 2 vol ethanol at room temperature, subsequently collected by centrifugation at $10^4$ x g for 10 min and washed twice by redissolving the DNA in distilled, sterile water and precipitating it again with ethanol. Finally, the DNA is redissolved in TE buffer. RNA is then removed by adding RNase A (20 µg/ml).

Example 1.4: Determination of heterologous hybridization conditions

High molecular weight DNA isolated from A.niger as described in Example 1.3 is digested with EcoRI and BamHI. The resulting fragments are separated by agarose gel electrophoresis, and transferred to nitrocellulose as described by Maniatis et al. (1982) pp. 383-389. Heterologous hybridization conditions are determined and hybridization experiments are performed as previously described by de Graaff et al. (1988). The hybridization conditions for screening the library and for hybriding Southern blots with the 1.8 kb EcoRI fragment of the yeast gene as a probe are: prehybridization in 6 x SSC, 0.1 % SDS, 0.05 % sodium pyrophosphate and 20 µg/ml denatured herring sperm DNA at 56 °C for 3-5 h, hybridization in 6 x SSC, 0.1 % SDS, 0.05 % sodium pyrophosphate and 20 µg/ml denatured herring sperm DNA at 56 °C for 15-18 hrs, followed by two washes in 5 x SSC, 0.1 % SDS at 56 °C and two washes in 2 x SSC at 56 °C. The filters are autoradiographed overnight at -70 °C using Konica X-ray films and Kodak X-Omatic cassettes with regular intensifying screens.

Example 1.5: Screening of the gene library with the radioactive 1.8 kb EcoRI fragment

The A. niger pki gene is isolated by heterologous hybridization using the 1.8 kb EcoRI fragment of pPYK1 as probe. A genomic library of A. niger N400, which is prepared according to the method described in EP-A-0278355, is screened with the 1.8 kb EcoRI fragment of pPYK1 by a hybridization procedure according to the method described in Example 1.4. The screening results in the isolation of positive clones. From the clones giving the strongest hybridization signals plasmid DNA is isolated by mini-prep-isolation (Maniatis. et al., 1982, pp.368- 369). Southern and restriction analysis is used to check up whether a clone contains the complete A. niger pki gene. The 5 kbp BglII/HindIII fragment of this clone and the 4.0 kbp BamHI/HindIII vector fragment of the E. coli vector pBR322 are isolated according to the LMP agarose method described in Example 1.1. The 5 kbp BglII/HindIII fragment is ligated with the 4.0 kbp BamHI/HindIII vector fragment of pBR322, resulting in the plasmid pGW1100, by the following procedure: 100 ng of the pBR322 fragment is mixed with 250 ng of said 5 kbp BglII/HindIII fragment and 4 µl 5 x ligation buffer (composition: 250 mM Tris/HCl pH 7.6; 50 mM $MgCl_2$; 50 mM DTT; 5 mM ATP; 5 mM spermidine; 50 µg/ml BSA) and 1 µl (1.2 U/µl) DNA ligase (BRL) is added to this mixture in a final volume of 20 µl. After incubation for 16 h at 14 °C the mixture is diluted to 100 µl with sterile water. 10 µl of the diluted mixture is used to transform competent E. coli JM101 cells, which are prepared according to the CM1, CM2 method described in the Pharmacia Manual for the M13 cloning/sequencing system. pGW1100 is isolated on a large scale (Maniatis, 1982, p. 86), purified by CsCl density gradient centrifugation and extraction with phenol, precipitated with ethanol and dissolved in TE buffer. The plasmid pGW1100 is further analyzed by restriction analysis and the orientation of the pki gene is determined by hybridization under conditions described in Example 1.4 using fragments containing the 5'- and 3'- end of the S. cerevisiae pyruvate kinase gene, a 1.0 kbp EcoRI/BglII and a 0.88 kbp EcoRI/BglII fragment, respectively, as probes.

Example 1.6: Sequence determination of the A. niger pyruvate kinase gene

The sequence of the A. niger pki gene, including the promoter region, the structural gene and the termination region, is determined by subcloning fragments from pGW1100 in M13mp18/mp19. For nucleotide sequence analysis suitable restriction fragments are isolated as described in Example 1.1 and are ligated with linearized in bacteriophage M13 mp18/19 RF DNA vectors (Messing, 1983; Norrander et al., 1983) as described in Example 1.5. The nucleotide sequences are determined by using the dideoxynucleotide chain-termination procedure (Sanger et al., 1977) as described in M13 Clonin/Dideoxy Sequencing Instruction Manual from BRL, pp. 50-73. The sequence determined is given in the sequence listing under SEQ ID NO. 1.

Example 2: Construction of expression vectors

Example 2.1: Introduction of a new restriction site at the translation initiation codon of the pyruvate kinase gene

Example 2.1.1: Preparation of uracil containing M13mp18-PK (BamHI-PvuII) template DNA

Bacteriophage M13mp18-PK (BamHI-PvuII) obtained in Example 1.6, is propagated by inoculating a 5 ml culture of E. coli JM101 in 2xYT nutrient medium at

$$O.D._{600\ nm}^{1\ cm} = 0.1$$

with a single plaque. After overnight growth at 37°C, the bacteria are removed by centrifugation (10,000 x g, 10 min), and the supernatant is used as a phage stock in preparing the uracil containing single stranded DNA template.

The uracil containing template is prepared according to Kunkel et al. (1985) and Ner et al., (1988) as follows: 10 ml 2xYT medium containing 5 mM uridine is inoculated with E. coli RZ1032 and the culture is grown at 37°C up to an

$$O.D._{550\ nm}^{1\ cm}$$

of 0.3. Of this culture 2.5 ml are diluted in 10 ml 2xYT medium containing 5 mM uridine. This culture is infected by the addition of 12 µl of the M13mp18-PK (BamHI-PvuII) containing supernatant prepared as described above. The infected culture is grown for 5 h at 37°C. After this growth period the bacteria are removed from the culture as described above, and the phage containing supernatant is used for preforming a second cycle of phage growth on E. coli RZ1032 as described before. In a third cycle of growth, 40 ml 2xYT medium containing 5 mM uridine are mixed with 10 ml of a culture of E. coli RZ1032 and 50 µl of the phage containing supernatant resulting from the second growth cycle are added. The bacteria are grown for 5 h at 37°C and the bacteria are removed from the supernatant by centrifugation as described above. The supernatant is centrifuged a second time before the phages are precipitated by the addition of 8 ml of 20 % polyethyleneglycol-6000/3 M NaCl. The phages are collected by centrifugation for 10 min at 10,000 x g. The resulting phage pellet is resuspended in 2.5 ml TE buffer. The proportion of uracil containing phages is determined by plating different dilutions of this phage solution on E. coli JM101 (non-permissive) as well as on E. coli RZ1032 (permissive). A ratio of non-uracil to uracil containing phages of 1 to $10^6$ is found. Single stranded M13mp18-PK (BamHI-PvuII) DNA is isolated from the phage solution by phenol- chloroform extraction, precipitated with ethanol as described in Example 1.1 and resuspended in 125 µl TE buffer.

Example 2.1.2: Introduction of an NsiI site at the translation initiation site of the A. niger pki gene by in vitro mutagenesis

A NsiI site is created at the translation initiation site of the pyruvate kinase gene by in vitro mutagenesis (Ti-Zi Su and M. Raafat El- Gewely, 1988) of the uracil containing single stranded M13mp18-PK (BamHI-PvuII) DNA described in Example 2.1.1. using the mutagenic oligonucleotide 5926 which has the nucleotide sequence with the SEQ ID NO. 2.

The oligonucleotide 5926 consists of 29 nucleotides the sequence of which is identical with the sequence around the translation initiation site of the pki gene except positions 12 to 14 of the oligonucleotide. The sequence of the nucleotides in positions 12 to 14, which in the original pki translational initiation region reads GCC, is CAT in the oligonucleotide. Therefore, the oligonucleotide has a NsI site in positions 9 to 14.

For the in vitro mutation of the pki gene 50 pmol of the oligonucleotide 5926 are phosphorylated at the 5' end (Maniatis, 1982) with 100 pmol ATP. This reaction is carried out for 30 min at 37°C with 10 U T4 polynucleotide kinase (BRL) in 50 µl kinase buffer as recommended by the supplier. The reaction is terminated by the addition of 2 µl of a 500 mM EDTA solution. The mixture is extracted with phenol and chloroform as described in Example 1.1.

0.2 pmol uracil-containing single-stranded M13mp18-PK (BamHI-PvuII) DNA prepared according to Example 2.1.1. is mixed with 0.5 pMol phosphorylated oligonucleotide 5926 in 20 mM Tris-HCl pH 7.5, 10 mM $MgCl_2$, 25 mM NaCl in a final volume of 10 µl. The mixture is incubated for 5 min at 65°C, slowly cooled to room temperature during 60 min and placed on ice for 15 min. Then 2 µl 500 µM dNTP's, 1.5 µl 10 mM ATP, 1 ml $T_7$ DNA polymerase (12 U/µl) (Pharmacia) and 1 µl T4 DNA ligase (1.2 U/µl) (BRL) are added to the mixture and this polymerization mixture is incubated for 15 min at 37°C. The reaction is terminated by heating at 65°C for 5 min. The polymerization mixture is then diluted to a final volume of 100 µl and aliquots of the diluted mixture are used to transfect competent E. coli JM101 (non-permissive) and E. coli RZ1032 (permissive) which are prepared as described in Example 1.5. The transfected cells are plated as described also in Example 1.5.

Example 2.1.3: Sequence analysis of the mutated M13mp18-PK (BamHI-PvuII)

Twelve plaques are picked from the plates with transformed E. coli JM101, phages are propagated with E. coli JM101 as a host and from each phage single stranded DNA is isolated as described in Example 2.1.1. The isolated single stranded DNA of these phages is analyzed by 'G-track' analysis, as described in BRL's M13 cloning and sequencing manual. Three of the phages with the predicted G pattern for the desired mutation are analyzed by sequence analysis, as described in Example 1.6. The phages comprise the predicted 1053 bp BamHI-PvuII fragment with the NsiI site at the translation initiation site of the pki gene. The mutated phages are named M13mp18-PK (BamHI-NsiI-PvuII).

Example 2.2: Construction of pPK-PLB

The 742 bp BamHI/NsiI-fragment containing the pki promoter region is isolated from the M13mp18-PK (BamHI-NsiI-PvuII) RF DNA, while a 2.6 Kb BamHI/NsiI-fragment, containing the structural gene for pectin lyase B (pel B), is isolated from plasmid pGW830 according to the method described in Example 1.1. Both fragments are ligated in a dephosphorylated pBR322-vector digested with BamHI (Fig. 4) as follows: 100 ng BamHI digested pBR322 DNA is mixed with 250 ng of the 2.6 kb pelB BamHI/NsiI-fragment and with 250 ng of the 742 bp pki BamHI/NsiI-fragment. The mixture is ligated as described in Example 1.5. Aliquots of the diluted ligation mixture are used to transform competent E. coli JM101 cells, prepared as described in Example 1.5. The transformation mixture is plated on LC-plates containing 50 $\mu$g/ml ampicillin and incubated at 37 °C over night. Transformants are picked from the plate and grown for 5 h at 37 °C in liquid LC medium containing 50 $\mu$g/ml ampicillin. Plasmid DNA is isolated from transformants by miniprep-isolation (Maniatis. et al., 1982, pp.368-369 ). A transformant containing a plasmid which reveals the expected fragments after digestion with BamHI, SphI, SmaI and with the combination of BamHI and NsiI is further analyzed by sequence analysis, using a pel B specific oligonucleotide as primer. The sequence corresponds to the predicted sequence of the fused gene pki-pel B. The plasmid is named pPK-PLB, and is propagated and purified as described in Example 1.5.

Example 3:

Example 3.1: Introduction of pPK-PLB in A.niger

The plasmid pPK-PLB, obtained in Example 2.2 is introduced in A. niger by cotransformation of A. niger N593 with plasmids pGW613 and pPK-PLB. pGW613 comprises the selective marker gene pyr A.

Protoplasts of A. niger N593 are prepared from mycelium by growing A. niger N593 on minimal medium supplemented with 0.5 % yeast extract, 0.2 % casamino acids, 50 mM glucose and 10 mM uridine for 20 h at 30 °C. The preparation of protoplasts of A. niger N593 and the transformation procedure is performed as described by Goosen et al., 1987. The resulting $PYR^+$ transformants are grown on said supplemented minimal medium and are analyzed for the expression of the pel B gene.

Example 3.2: Analysis of PK-PLB transformants of A. niger N593

A. niger transformants obtained in Example 3.1 are analyzed for the formation of the pel B gene product, the PLB protein. They are selected and grown for 18 h on medium containing 10 g 72 %-esterified pectin, 7.5 g $NH_4NO_3$, 0.5 g KCl, 0.5 g $MgSO_4$, 1.5 g $KH_2PO_4$, 0.5 g casamino acids, 0.5 g yeast extract and 0.5 ml of a stock solution of spore elements, $H_2O$ ad 1 l, pH 6.0. The stock solution of spore elements consists per liter of 10 g EDTA, 4.4 g $ZnSO_4.7H_2O$, 1.01 g $MnCl_2.4H_2O$, 0.32 g $CoCl_2.6H_2O$, 0.315 g $CuSO_4.5H_2O$, 0.22 g $(NH_4)_6Mo_7O_{24}.4H_2O$, 1.47 g $CaCl_2.2H_2O$ and 1.0 g $FeSO_4.7H_2O$. After growth the mycelium is removed by filtration and the culture filtrate is analyzed by SDS-polyacrylamide gel electrophoresis, using a gel containing 10 % acrylamide. The PLB protein is detected by Western-blotting assay (as described in the manual for the 2117 multiphor II semy-dry blot apparatus of LKB) using polyclonal antibodies from rabbits raised against PLII (van Houdenhoven, 1975) and goat-anti-rabbit antibody conjugated to alkaline phosphatase (Bio Rad) according to the manufactors instructions. Of the transformants analyzed, about 70 % produce the PLB protein. One transformant is selected for further work. Hereinafter, the transformant is referred to as A. niger/PK-PLB.

Example 3.3.: Northern analysis of the pPK-PLB transformant of A. niger

The A. niger transformant A. niger/PK-PLB selected according to Example 3.2 is analyzed for the formation of pelB specific mRNA. The transformant is grown for 16 h at 30 °C on a medium as described in Example 3.2 except for the carbon source which is 2% (w/v) D-glucose. After harvesting, the mycelium is quickly dried by pressing it between sheets of paper, and is immediately immersed in liquid nitrogen to grind it. One g of powdered mycelium is then suspended in 3 ml buffer (4.0 M guanidinium thiocyanate, 50 mM Tris-HCl pH 7.5, 10 mM EDTA pH 7.5, 1% $\beta$-mercaptoethanol, 2% sodium lauryl sarcosinate), and vortexed for 1 min. After centrifugation (10 min, 10 000xg at room temperature) the supernatant is recovered. To each 2.5 ml supernatant 1 g CsCl is added. The samples are subsequently layered onto a cushion of 5.7 M CsCl, 0.1 M EDTA (pH 7.5) in a SW50 Beckman ultracentrifuge tube. Centrifugation is performed for 16 h at 30 000 rpm at 20 °C using a Beckman SW50 rotor. RNA pellets are dissolved in sterile distilled water. The amount of RNA of the samples is adjusted to approx. equal concentrations after agarose gel electrophoresis. Amounts of approx. 25 µg of RNA are run using glyoxal denaturation [Maniatis et al. (1982) Molecular cloning: A laboratory manual. Cold Spring Harbor Laboratory, New York. Page 200)]. The RNA is blotted on Gene-bind (Pharmacia) using 10•SSC as transfer buffer and baked for 1 h at 80 °C. Hybridization is carried out for 16 h in the hybridization buffer described by Church and Gilbert [Proc. Natl. Acad. Sci. USA 81:1991-1995(1984)] (1% BSA, 1 mM EDTA, 0.5 M $NaP_i$ pH 7.2, 7% SDS) at 60 °C using as probe the radioactively labelled 2.8 kbp XhoI fragment on which the pelB gene is situated. The blots are washed twice for 30 min with 2•SSC, 0.1% SDS and then twice for 30 min with 0.2•SSC, 0.1% SDS at 60 °C. Exposure of the blots is carried out over night at -70 °C using Konica X-ray film and intensifying screens. This analysis indicates a strong pelB specific mRNA signal in the transformant, and no signal in the control strain A. niger N400 grown similarly.

Example 3.4.: Production, purification and characterization of pectin lyase B (PL B) from the A. niger/PK-PLB

Example 3.4.1.: Culture conditions to prepare pectin lyase B and isolation of the enzyme

Spores of A. niger/PK-PLB are used to inoculate 600 ml of culture medium to a density of $7x10^6$ spores/ml. The medium has the following composition: 20 g glucose, 7.5 g $NH_4NO_3$, 0.5 g KCl, 0.5 g $MgSO_4.7H_20$, 15 g $KH_2PO_4$, 5 g yeast extract, 0.5 g ribonucleic acids, 0.5 ml of a stock solution of spore elements, $H_2O$ ad 1 l, pH 6.0. The stock solution of spore elements consists per liter of 10 g EDTA, 4.4 g $ZnSO_4.7H_2O$, 1.01 g $MnCl_2.4H_2O$, 0.32 g $CoCl_2.6H_2O$, 0.315 g $CuSO_4.5H_2O$, 0.22 g $(NH_4)_6Mo_7O_{24}.4H_2O$, 1.47 g $CaCl_2.2H_2O$ and 1.0 g $FeSO_4.7H_2O$. The culture is grown for 3 h at 30 °C in a New Brunswick orbital shaker and then transferred to a 10 l flask containing 4 l of the same medium. The culture is aerated using a sparger at the bottom of the flask to distribute the compressed air and agitated the culture. Growth is continued for 16 h at 30 °C. After this growth period the pH of the medium is 5.8. The mycelium is removed by filtration and PLB is isolated from the culture filtrate by following procedure:

The enzyme is recovered almost quantitatively by ammonium sulphate precipitation (95 % saturation) at 0 °C followed by centrifugation (15 min., 8500 x g). The precipitated enzyme is dissolved in 50 mM sodium phosphate buffer pH 6.0, dialyzed against the same buffer, and stored at -20 °C. The pectin lyase activity is assayed at 25 °C using a final concentration of 0.3 % (w/v) pectin (degree of esterification 94.6 %) in 50 mM Tris/HCl buffer, 1 mM $CaCl_2$, pH 8.5 using the spectrophotometric procedure described by van Houdenhoven, 1975, p.11.

Analysis of the culture filtrate in SDS-polyacrylamide electrophoresis and Western-blotting indicates a predominant protein band corresponding with PL B and almost pure enzyme in the precipitated and dialyzed fraction. The specific pectin lyase activity in the culture filtrate is 48 U/mg protein and in the precipitated and dialyzed fraction is 216 U/mg protein and the protein concentration is 380 mg and 82.6 mg, respectively, as determined by the Pierce BCA assay.

Example 3.4.2.: Properties of the PL B protein

The apparent molecular mass of the PLB as isolated in Example 3.1.3. is 39.5 kDa as is determined by SDS-polyacrylamide gel electrophoresis using 10 % gels. The purified enzyme is stable in 50 mM sodium phosphate buffer pH 6.0, but is inactivated slowly at higher pH e.g. in 50 mM Tris/HCl buffer pH 7.5. This inactivation is reversible and the activity can be largely regained by dialyzing the enzyme solution against phosphate buffer pH 6.0. The PLB prepared in Example 3.1.3. is a typical endo-pectin lyase, as can be

concluded from the oligomeric breakdown products which arise from highly esterified pectin (d.e. 94.6%). The enzyme prefers highly esterified pectin as shown in Table 1.

Table 1

| Activity of PL B on apple pectin with different degrees of esterification.[a] | |
|---|---|
| degree of esterification of pectin substrate (%) | relative enzymatic activity of PLB (%) |
| 94.6 | 100 |
| 72.8 | 29 |
| 61.2 | 20 |
| 50.6 | 11 |
| 34.8 | 5 |

[a]Assay conditions: 0.3 % (w/v) pectin in 50 mM Tris/HCl buffer pH 8.5 containing 0.5 M NaCl at 25°C.

The Michaelis-Menten parameters for PL B have also been determined, using enzyme which is stored in sodium phosphate buffer pH 6.0. The activity was assayed at 25°C in 50 mM Tris/HCl buffer pH 8.5 in the presence of 0.5 M NaCl, using different concentrations of highly esterified pectin (d.e. 94.6 %). A Km value of 9 mM (on monomer basis) and a turnover number of 51500 is found.

Example 4: Expression of human hybrid interferon BDBB under the control of the pki promoter

Example 4.1: Construction of plasmid pGII-IFN AM119 precursor

Plasmid pGW1800, which is isolated from E. coli JM109/pGW1800 (DSM 5505), is digested with EcoRI and treated with T4 polymerase as below. Religation of this DNA and transformation of E. coli DH5αF' allows the isolation of a plasmid pGW1800-E, which is the same as pGW1800, except that the EcoRI cleavage site is deleted.

Plasmid pGW1800-E is digested with BglII and treated with bacterial alkaline phosphatase (BRL) in the presence of 50 mM Tris-HCl pH 8.0 and 50 mM NaCl for 1 h at 65°C. The alkaline phosphatase is inactivated by digestion with proteinase K (Boehringer Mannheim) and phenol extracting. The DNA is subsequently ethanol precipitated, dried and redissolved in water. Then the sticky ends are filled in with T4 DNA polymerase as below.

Plasmid pJDB207-IFN AM119 (EP 205 404) is digested with HindIII and ClaI. The sticky ends of these linear fragments are filled in with T4 DNA polymerase (Boehringer Mannheim) in the presence of 0.1 mM each of dCTP, dGTP, dATP and dTTP plus 67 mM Tris-HCl pH 7.5, 6.7 mM $MgCl_2$, 16.7 mM $(NH_4)_2SO_4$ and 5 mM DTT for 30 min at 37°C. The reaction is stopped by heating to 65°C for 5 min. The fragments are separated in a 0.8 % low gelling temperature agarose (BioRad) gel and the 1 kbp fragment, which comprises the IFN AM119 coding region, was excised and the DNA purified on an Elutip D (Schleicher & Schüll) column, and ethanol precipitated (Schmitt & Cohen, 1983).

100 µg of the IFN AM119 fragment and the pGW1800-E vector prepared above are ligated together in 5 µl of 20 mM Tris-HCl pH 7.5, 10 mM $MgCl_2$, 10 mM DTT, 1 mM ATP and 1 unit of T4 DNA ligase (Boehringer Mannheim) for 2 h at room temperature. This mixture is transformed into competent E. coli DH5αF' cells. Ampicillin resistant transformants are screened by restriction digestion of their plasmid DNA to identify those that carry the plasmid pGII-IFN AM119 precursor.

Example 4.2: The generation of pGIIss-IFN AM119 using PCR

The PCR method followed is as described by R.M. Horton et al. (1989). pGW1800 is linearised, by XbaI digestion, and precipitated with ethanol. After resuspension in water, 100 µg of this DNA is subjected to amplification by polymerase chain reaction (PCR) using oligonucleotides A and C (SEQ ID NO. 5 and 6, respectivley) in an automated thermal cycler for 25 cycles (each consisting of 1 min at 94°C, 2 min at 40°C, and 3 min at 72°C) followed by 10 min at 72°C. 100 pM of each oligonucleotide and 0.5 µl of Taq polymerase (Perkin Elmer Cetus) are used for each reaction in a volume of 100 µl using the reaction buffer recommended by the supplier. This gives DNA 2 (SEQ ID NO. 7).

Similarly pJDB207-IFN AM119 linearised with BamHI and subjected to PCR with either oligonucleotides D (SEQ ID NO. 8) and F (SEQ ID NO. 9) gives DNA 3 (SEQ ID NO. 10).

These reaction mixtures are precipitated with ethanol, redissolved in water and an aliquot is checked on a gel to determine the concentration of the DNA fragments in the mixtures.

Using the same conditions as above DNA 2 and 3 are subjected to PCR with oligonucleotides A and F to give a DNA sequence which comprises a perfect in frame fusion of the PGII signal sequence linked to a PGII promoter fragment with the coding region for mature hybrid interferon BDBB.

The BamHI-EcoRI fragment of this DNA, which contains the perfect in frame fusion is ligated into BamHI-EcoRI-cut pGII-IFN AM119 precursor to generate plasmid pGIIss-IFN AM119. The part of the sequence of pGIIss-IFN AM119 comprising the PGII signal sequence, the BDBB hybrid IFN AM119 gene, the yeast pH05 terminator is depicted under SEQ ID NO. 11.

Example 4.3.: Construction of plasmid pPKI-IFN-1

Two µg of pGW1100 is opened with restriction endonuclease NsiI at a site at the 3' end of the pki gene. This DNA is treated with T4 polymerase for 30 min at 37 °C in a 20 µl reaction mixture containing: 2 µg of DNA, 67 mM Tris/HCl pH 8.8, 6.7 mM $MgCl_2$, 16.7 mM $(NH_4)_2SO_4$, 5 mM Dithiothreitol, 50 µM each of dGTP, dATP, dCTP and dTTP, 1 U T4 polymerase. After ethanol precipitation 100 ng of this DNA is ligated with 10 pM of unphosphorylated EcoRI oligonucleotide linker with the sequence 5' GGAATTCC at room temperature for 2 h in a 5 µl reaction mixture containing: 100 ng DNA, 10 pM linker, 20 mM Tris/HCl pH 7.5, 10 mM $MgCl_2$, 10 mM Dithiothreitol, 1 mM ATP and 1 U ligase. This mixture is transformed into E. coli DH5αF' and selected for plasmid containing cells on 2xYT plus 50 µg/ml Ampicillin. After making DNA from 18 colonies and screening them by restriction digest, plasmid pGW1100-E comprising the EcoRI linker sequence and lacking the NsiI site is identified.

Plasmid pGIIss-IFNAM119 which is prepared according to Example 4.2 contains the Aspergillus niger PGII promoter and signal sequence fused to a IFN gene that is followed by the Sacharomyces cerevisiae PH05 terminator and the PGII terminator. This plasmid is cut with PstI at the end of the PH05 promoter and blunt ended with T4 polymerase and ligated to a SphI oligonucleotide linker with the sequence 5' GGCATGCC and transformed into E. coli DH5α exactly as above, to create plasmid pGIIss-IFNAM119Sph.

50 ng each of the following four purified fragments are ligated together as above:

- The 742 base pair BamHI/NsiI-fragment, containing the pki promoter region, from the mutated M13mp18-PK (BamHI-NsiI-PvuII) RF DNA. The NsiI site is removed by treating the NsiI cut plasmid with T4 polymerase before cutting with BamHI.
- The approximately 1.1 kbp BamHI/SphI fragment, containing the 3' end of the PGII promoter plus the PGII signal sequence plus the IFN gene, from PGIIss-IFNAM119SRh. The BamHI site was filled in with T4 polymerase before cutting with SphI.
- The 417 bp SphI/EcoRI fragment, containing the terminator region of pki gene, from pGW1100-E.
- The approximately 2.8 kb fragment of BamHI/EcoRI cut pTZ18R (Pharmacia). After transforming into E. coli DH5αF' and screening plasmid PKI-IFN-1 is identified.

Example 4.4.: Mutagenesis of pPKI-IFN-1 to create pPKI-IFN-2 and pPKIssIFN-2

Plasmid pPKI-IFN-1 was transformed into a $dut^-ung^-$ E. coli strain BW313. This was superinfected with M13K07 to yield single stranded uracil-substituted phage from the plasmid. This phage DNA was prepared and mutagenised as described above with the two different oligonucleotides IFN-1 and IFN-2, respectively, the sequences of which are depicted in the sequence listing with SEQ ID NO. 12 and 13, respectively. Mutagenisation with oligonucleotide IFN-1 yields pPKI-IFN-2, and with IFN-2 yields pPKIssIFN-2. Both plasmids comprise perfect in frame fusions. pPKI-IFN-2 has the pki promoter fused to a methionine start codon and then the rest of the IFN gene and pPKIssIFN-2 has the pki gene fused to the PGII signal sequence followed by the IFN gene. The nucleotide sequences of the regions of pPKI-IFN-2 and pPKIssIFN-2 extending from the pki promoter up to the terminator region are depicted under SEQ ID NO. 14 and 15, respectively.

Example 4.5: Transformation of the PKI-IFN expression plasmid into Aspergillus niger

The plasmids pPKI-IFN-2 and pPKIssIFN-2 are cotransformed into A. niger N593 using the A. niger pyrA gene as a selectable marker on the plasmid pGW613 as described above.

Example 4.6: Analysis of transformants

The transformants from example 4.5 are analysed for the production of IFN by Western analysis as described in Example 3.2 but using an antibody raised against IFN (instead of an anti-PLII antibody).

Deposited microorganisms

E. coli DH5αF'/pGW1100 was deposited as No. DSM 5747 on January 18, 1990 and A. niger N593 as No. DSM 5756 on January 26, 1990 according to the Budapest Treaty with the Deutsche Sammlung von Mikroorganismen und Zellkulturen, Mascheroder Weg 1b, D-3300 Braunschweig,

**References**


- Bolivar, F., Rodriguez, R.L., Greene, P.J. Betlach, M.C., Heyneker, H.L., Boyer, H.W., Crosa, J.W., and S. Falkow (1977) Construction and Characterization of new cloning vehicles II: A multipurpose cloning system. Gene 2: 95.
- BRL: M13 Cloning/Dideoxy Sequencing Instruction Manual
- Burke, R.L., Tekamp-Olson, P. and Najarian, R. (1983): The isolation, characterization and sequence of the pyruvate kinase gene of Saccharomyces cerevisiae. J. Biol. Chem., 258: 2193-2201.
- Dotto, P.D. and Zinder, N.D. (1984). Nature 311: 279
- Goosen, T., Bloemheuvel, G., Gysler, C., de Bie, D.A., van den Broek, H.W.J. and Swart, K. (1987) Transformation of Aspergillus niger using the homologous orotidine-5'-phosphate-decarboxylase gene, Curr. Genet., 11: 499-503.
- de Graaff, L.H., van den Broek, H.W.J. and Visser, J. (1988): Isolation and transformation of the Aspergillus nidulans pyruvate kinase gene. Curr. Genet., 13: 315-321.
- de Graaff, L.H. (1989). The structure and expression of the pyruvate kinase gene of Aspergillus nidulans and Aspergillus niger. Ph. D. Thesis. Agricultural University of Wageningen. The Netherlands.
- Horton, R.M. Hunt, H.D. Ho, S.N., Pullen, J.K., and Pease, C.R. (1989). Engineering hybrid genes witout the use of restriction enzymes: gene splicing by overlap extension. Gene 77: 61-68.
- Kunkel, T. (1985): Rapid and efficient site-specific mutagenesis without phenotypic selection, Proc. Natl. Acad. Sci. USA, 82: 488-492.
- Maniatis T., E. F. Fritsch, J. Sambrook (1982): Molecular cloning, a laboratory manual; Cold Spring Harbor Laboratory, New York.
- Mead et al. (1986). Protein Engeneering 1:67.
- Messing, J. (1979). A multipurpose cloning system based on single stranded DNA bacteriophage M13. Recomb. DNA Techn. Bull 2 (2): 43.
- Messing, J. (1983): New M13 vectors for cloning. Methods in Enzymol., 101C: 20-78
- Ner, Sarbjit S., Goodin, D.B. and Smith, M. (1988): A simple and efficient procedure for generating random point mutations and for codon replacements using mixed oligodeoxynucleotides, DNA, Vol. 7, No. 2: 127-134.
- Norrander, J., Kempe, T. and Messing, J. (1983): Construction of improved M13 vectors using oligodeoxynucleotide directed mutagenesis. Gene, 26: 101-106
- Pharmacia: Manual for the M13-cloning/sequencing system, including M13mp18/M13mp19.
- Peden, K.W.C. (1983). Revised sequence of the tetracyclin-resistance gene of pBR322. Gene 22, 277-280.
- Sanger, F., Nickelen, S. and Coulson, A.R. (1977): DNA sequencing with chain terminaing inhibitors; Proc. Natl. Acad. Sci. USA, 74: 5463-5467.
- Schmitt, J.J and Cohen, B.N. (1983). Quantitative isolation of restriction fragments from low-melting agarose by Elutip-α affinity chromatography. Analytical Biochemistry 133: 462-464.
- Slater, R.J. (1985): The extraction of total RNA by the detergent and phenol method. In: Walker J.M. (ed), Methods in Molecular Biology, vol 2, 1985, Nucleic acids, Humana press, Cliftar, New Yersey, pp. 101-108
- Sutcliffe, J.G. (1979). Complete nucleotide sequence of the Escherichia coli plasmid pBR322. Cold Spring Harbor Symposia Quant. Biol. 43: 77-90.
- Ti-zi Su and M. Raafat El-Gewely (1988): A multisite-directed mutagenesis using $T_7$-polymerase: application for reconstructing a mammalian gene, Gene 69: 81-89.
- van Houdenhoven, F.E.A. (1975): Ph.D. thesis, Agricultural University, Wageningen, The Netherlands.

- Vishniac, W. and Santer (1957). Bacteriol. Rev. 21: 195-213.

Sequence listing

SEQ ID NO. 1

SEQUENCE TYPE: Nucleotide with corresponding protein

SEQUENCE LENGTH: 3605 bp
STRANDEDNESS: double
TOPOLOGY: linear
MOLECULE TYPE: genome
ORIGINAL SOURCE ORGANISM: Aspergillus niger N400
IMMEDIATE EXPERIMENTAL SOURCE: Plasmid pGW1100 from E. coli DH5αF'/pGW1100
FEATURES: from 1 to 1041 bp pki promoter region
from 831 to 835 bp putative CAAT box
from 928 to 933 bp putative TATA box
from 849 to 1040 bp CT rich region
from 1042 to 3096 bp coding region for pyruvate kinase
from 1166 to 1266 bp intron 1
from 1312 to 1370 bp intron 2
from 1418 to 1472 bp intron 3
from 1545 to 1606 bp intron 4
from 1729 to 1828 bp intron 5
from 2663 to 2711 bp intron 6
from 2794 to 2851 bp intron 7

```
AAACCTCCAA ATGGAAGAGA AAACCTCCGA GTACTTACTT    40
AGGGTCCCTG TCTACTGGCC AGAGTCTCGT CCTCATTACT    80
ATGATTAATT ACCCACTGGA CAAAAAAATA AAATAAAATA   120
AAAATAAAAA GGGAGACAGC TTCTCCATAA CTGGCAACTG   160
GGTCCGTCCG AGCAGAGCAA AATTCAGCCT TATGGGTTCC   200
GATGGAGTCA GGGAAATAGT TCTTGCGAAG GGCATTGGGC   240
TTTTTTGCGA GGAGAAAATT CAGCACCGAC AAAGCATCCA   280
AATCCACCTC GCTAGGAGAG AATGGATCCG CGACGATGTG   320
GGGTCAACTG GACAGAGTGA GAGGGTATCA TGTGGTCCTG   360
CCAGATACTT CGCAGAATGT TGTGTGGGTG TCTGATTGTG   400
```

```
GCTTGGGCGT GAATTGCTTT TGGTCTTCCC AACCAATTAT          440
TATGCAAGCC GCCGAAGAAA GCCCGAAAAG CCCCGAAGGA          480
AAGGGCCCGA AGAAGGAAGG GAAAAAAGCC GCCGAACCCC          520
GGGCCGAAAA CCCTGGCCGA ACAAGGAAAA AAACCGAACG          560
AAGCAACAAG GGGAACCAAC AACAAAAAAA AAGAACAAAA          600
CAAGGAAAAG GGGAACAACC AACAAAAAAA TGAAGTTTGA          640
AAACCAACCA AAAGGCCCGG GGGGAAAAAA AAGTCATTAA          680
TAATGGGAAT TATGTAGGCG ATGGGAAGTG TGATTGTAAC          720
TACTCCGTAG CTGGAGGCAC AACTAACAAG CCAGCTCTCA          760
ACCCGCGGGG AACCGACCGA CAGAAAAAAG CGTCCCAAAG          800
CAGGAATCCC ACCAAAAAGG GCCGATCCAG CCAATCACCG          840
CCGCCAACAT TTTTCCTTCC CGGGCACCCC TCCTCTAGTC          880
CACCATCTCT CTCTTCTCTC GCTCACCGGC CCCGTCTTTT          920
CCTTCCCTAT TATCTCTCCC TCTTCTCCTC CCTTCTCTCC          960
CTCCATTCTT TCTCCCATCT TCATCAATCC CTTCTCTTCT         1000
GTCTTCCCCC CCGGTTCAGT AGAGATCAAT CATCCGTCAA         1040
G ATG GCC GCC AGC TCT TCC CTC GAC CAC CTG AGC AAC   1077
  Met Ala Ala Ser Ser Ser Leu Asp His Leu Ser Asn
                    5                  10

CGC ATG AAG TTG GAG TGG CAC TCC AAG CTC AAC ACT     1113
Arg Met Lys Leu Glu Trp His Ser Lys Leu Asn Thr
        15                  20

GAG ATG GTG CCC TCC AAG AAC TTC CGC CGC ACC TCC     1149
Glu Met Val Pro Ser Lys Asn Phe Arg Arg Thr Ser
 25                  30                  35

ATC ATC GGA ACC ATC GGTACGTTCC TGCCAGTTGT GTCGTTGCGA  1194
Ile Ile Gly Thr Ile
            40

TTGCCATCTC TTGATGAGGA CCTCGCACCC CGCACTTGAC          1234
CTCATCCGAG CTAACCTGCG ATTTTTCTTC AG GC CCC AAG ACC   1277
                                    Gly Pro Lys Thr
                                                 45
```

```
AAC TCC GTG GAG AAG ATC AAC TCT CTC CGC ACT            1310
Asn Ser Val Glu Lys Ile Asn Ser Leu Arg Thr
                    50                  55

GGTACGAGCG ATAAAACATA TAACCTCTGG GAGTTATCAA            1350
TCAGCTGACT AGCTTGCAG CC GGT CTT AAC GTT GTT CGC ATG   1393
                    Ala Gly Leu Asn Val Val Arg Met
                                    60

ACC TTC TCC CAC GGT TCT TAT GAG GTAAGAAGGG AACCCATCCG 1437
Asn Phe Ser His Gly Ser Tyr Glu
 65                  70

TGCATTGGCA CATGACGATA TGCTGACCCG CCCAG TAC CAC CAA    1481
                                      Tyr His Gln
                                               75

TCT GTT ATC GAC AAC GCC CGC GAG GCC GCC AAG ACC        1517
Ser Val Ile Asp Asn Ala Arg Glu Ala Ala Lys Thr
                    80                  85

CAG GTC GGA CGT CCT CTC GCC ATT GCT CTT GAT ACC        1553
Gln Val Gly Arg Pro Leu Ala Ile Ala Leu Asp Thr
            90                  95

GTAAGTTCGG GTCCCTTGGC TGGTCGCGAT CCTCCAAATT            1593
AACTCCTCTG TAG AAA GGA CCC GAG ATC CGT ACC GGA         1630
               Lys Gly Pro Glu Ile Arg Thr Gly
               100                 105

AAC ACC CCC GAT GAT AAG GAT ATC CCT ATC AAG CAG        1666
Asn Thr Pro Asp Asp Lys Asp Ile Pro Ile Lys Gln
        110                 115
```

```
GGC CAC GAG CTC AAC ATC ACC ACC GAC GAG CAA TAT        1702
Gly His Glu Leu Asn Ile Thr Thr Asp Glu Gln Tyr
120                 125                 130

GCC ACC GCC TCC GAC GAC AAG AAC AT GTAAGATTCC          1738
Ala Thr Ala Ser Asp Asp Lys Asn Met
            135                 140

TCCCCGCGTC CTTCCGATCT TGCCAGTGGA TTCGGGAGAC            1778
CAGCGCAGAT GTAGTCTGTG CATAGACTCC GCTGACAAGT            1818
CGGATTGTAG G TAC CTC GAC TAC AAG AAC ATC ACC AAG       1856
             Tyr Leu Asp Tyr Lys Asn Ile Thr Lys
                             145

GTG ATC TCT CCT GGC AAG CTC ATC TAT GTT GAT GAC        1892
Val Ile Ser Pro Gly Lys Leu Ile Tyr Val Asp Asp
150                 155                 160

GGT ATC CTT TCC TTC GAG GTC CTC GAA GTC GTA GAT        1928
Gly Ile Leu Ser Phe Glu Val Leu Glu Val Val Asp
            165                 170

GAC AAG ACC ATC CGC GTC CGG TGC TTG AAC AAC GGC        1964
Asp Lys Thr Ile Arg Val Arg Cys Leu Asn Asn Gly
    175                 180                 185

AAC ATC TCT TCC CGC AAG GGT GTT AAC TTG CCC GGC        2000
Asn Ile Ser Ser Arg Lys Gly Val Asn Leu Pro Gly
                190                 195

ACT GAC GTT GAC CTC CCC GCC CTT TCC GAG AAG GAC        2036
Thr Asp Val Asp Leu Pro Ala Leu Ser Glu Lys Asp
        200                 205
```

```
ATT GCC GAT CTC AAG TTC GGT GTT AGG AAC AAG GTC        2072
Ile Ala Asp Leu Lys Phe Gly Val Arg Asn Lys Val
210                 215                 220

GAC ATG GTC TTC GCT TCT TTC ATC CGC CGC GGT AGC        2108
Asp Met Val Phe Ala Ser Phe Ile Arg Arg Gly Ser
            225                 230

GAC ATT CGC CAC ATC CGT GAG GTT CTG GGT GAG GAG        2144
Asp Ile Arg His Ile Arg Glu Val Leu Gly Glu Glu
    235                 240                 245

GGC AAG GAG ATC CAG ATC ATT GCC AAG ATT GAG AAC        2180
Gly Lys Glu Ile Gln Ile Ile Ala Lys Ile Glu Asn
                250                 255

CAG CAG GGT GTC AAC AAC TTC GAC GAG ATC CTC GAA        2216
Gln Gln Gly Val Asn Asn Phe Asp Glu Ile Leu Glu
        260                 265

GAG ACT GAC GGT GTC ATG GTT GCC CGT GGT GAC CTT        2252
Glu Thr Asp Gly Val Met Val Ala Arg Gly Asp Leu
270                 275                 280

GGT ATC GAG ATC CCC GCC CCC AAG GTC TTC ATC GCC        2288
Gly Ile Glu Ile Pro Ala Pro Lys Val Phe Ile Ala
            285                 290

CAG AAG ATG ATG ATC GCC AAG TGT AAC ATC AAG GGT        2324
Gln Lys Met Met Ile Ala Lys Cys Asn Ile Lys Gly
    295                 300                 305

AAG CCC GTC ATC TGT GCC ACT CAG ATG CTC GAG TCC        2360
Lys Pro Val Ile Cys Ala Thr Gln Met Leu Glu Ser
                310                 315
```

```
ATG ACA TAC AAC CCT CGT CCT ACT CGT GCC GAG GTG        2396
Met Thr Tyr Asn Pro Arg Pro Thr Arg Ala Glu Val
        320                 325

TCC GAT GTT GCC AAC GCC GTC CTT GAC GGT GCC GAC        2432
Ser Asp Val Ala Asn Ala Val Leu Asp Gly Ala Asp
330                 335                 340

TGT GTC ATG CTG TCG GGA GAG ACC GCC AAG GGT AAC        2468
Cys Val Met Leu Ser Gly Glu Thr Ala Lys Gly Asn
            345                 350

TAC CCC AAC GAG GCC GTC AAG ATG ATG TCC GAG ACC        2504
Tyr Pro Asn Glu Ala Val Lys Met Met Ser Glu Thr
    355                 360                 365

TGC CTG CTC GCC GAG GTT GCC ATC CCC CAC TTC AAT        2540
Cys Leu Leu Ala Glu Val Ala Ile Pro His Phe Asn
                370                 375

GTG TTC GAT GAG CTC CGC AAC CTT GCT CCT CGC CCC        2576
Val Phe Asp Glu Leu Arg Asn Leu Ala Pro Arg Pro
     380                 385

ACC GAC ACT GTC GAG TCC ATC GCC ATG GCT GCC GTT        2612
Thr Asp Thr Val Glu Ser Ile Ala Met Ala Ala Val
390                 395                 400

AGC GCC AGT CTG GAA CTC AAC GCT GGT GCC ATT GTC        2648
Ser Ala Ser Leu Glu Leu Asn Ala Gly Ala Ile Val
            405                 410

GTC TTG ACT ACC AG GTGAGTTGTA AATATCCAGA TGGGTAGGAT    2692
Val Leu Thr Thr Ser
    415
```

```
GATTGTCGAC AGATCGCAGC GGT AAA ACT GCT CGC TAC CTT      2733
                      Gly Lys Thr Ala Arg Tyr Leu
                          420                 425

TCC AAG TAC CGC CCC GTC TCG CCC ATT GTC ATG GTT        2769
Ser Lys Tyr Arg Pro Val Cys Pro Ile Val Met Val
                430                 435

ACC CGT AAC CCC GCT GCC TCC CGG GTAAGTCGAG             2803
Thr Arg Asn Pro Ala Ala Ser Arg
        440                 445

AAGCGTAGTG TTGTTTCGAG AGGTCGTGTG CTAACGTGTT            2843
GAATCCAG TAC TCT CAC CTG TAC CGT GGT GTC TGG CCC       2883
         Tyr Ser His Leu Tyr Arg Gly Val Trp Pro
                         450                 455

TTC CTC TTC CCC GAG AAG AAG CCC GAC TTC AAC GTC        2919
Phe Leu Phe Pro Glu Lys Lys Pro Asp Phe Asn Val
                460                 465

AAG GTC TGG CAG GAG GAT GTT GAC CGC CGT CTC AAG        2955
Lys Val Trp Gln Glu Asp Val Asp Arg Arg Leu Lys
        470                 475

TGG GGT ATC AAC CAC GCT CTT AAG CTC GGC ATC ATC        2991
Trp Gly Ile Asn His Ala Leu Lys Leu Gly Ile Ile
480                 485                 490

AAC AAG GGT GAC AAC ATC GTC TGT GTC CAG GGA TGG        3027
Asn Lys Gly Asp Asn Ile Val Cys Val Gln Gly Trp
            495                 500

CGC GGC GGT ATG GGC CAC ACC AAC ACC GTC CGT GTG        3063
Arg Gly Gly Met Gly His Thr Asn Thr Val Arg Val
    505                 510                 515
```

```
GTC CCT GCT GAG GAG AAC CTT GGC CTG GCT GAG TAA          3099
Val Pro Ala Glu Glu Asn Leu Gly Leu Ala Glu
                520                 525

ATGCAAAAGC AGTCTGGCAT GCCACCGGTT GGTGGATGAC              3139
ACGGTAACGA CAGCGATTGG ATAGAAAGCG TCAAGGGTGT              3179
GTCTCTGGAT ATCGTAGACC GGTCTCGCCG CCATGCGATG              3219
ACTCAGGTAG TCTCCCCGCG GGCAGGCTGC TTGTGTCTTC              3259
ACTGCGAGAC TCGTTAGTCG GGGCAGGACG AGGAGCACCA              3299
GGAGTGCGCA CTTGCGTCTA CCACCACATT CACTTTGAGC              3339
CCGAAACGCG CTGGGGGAAG ACACACACCT TGGGAACGTA              3379
ATGTGTATGT ATCTTGATTT GACGTTAGTT AGCCAGCCAT              3419
GTTTGGTGGA CTTTGCTGCG ATCAAAAGCT AGAATTTAAT              3459
GAGTTGTTCA CAATGCCTCG ATGAGATAAA GCACAGCATC              3499
CTGAGTGTCA GTCAGTATGA TTTGTTAGTG GAAATGCATT              3539
CCGAATCCCT TGAGGATCAG TTTCTGAAGG TAAACTCATC              3579
ACCCCCAAGC TGGCTATATA GAAACC                             3605
```

SEQ ID NO. 2

SEQUENCE TYPE: Nucleotide

SEQUENCE LENGTH: 21 bases

STRANDEDNESS: single

TOPOLOGY: linear

MOLECULE TYPE: synthetic oligonucleotide

FEATURES: from 8 to 13 NsiI site
from 1 to 7 complementary to A. niger pki nucleotides 1054 to 1048
SEQ ID
NO. 1)
from 14 to 21 complementary to A. niger pki nucleotides 1041 to 1034
(SEQ ID
NO. 1)

PROPERTIES: Mutagenic oligonucleotide 5926 for the introduction of a NsiI site in positions 1042 to 1047 of the A. niger pki gene.

```
AGCTGGCATG CATCTTGACG G                                  21
```

SEQ ID NO. 3

SEQUENCE TYPE: Nucleotide with corresponding polypeptide

SEQUENCE LENGTH: 821 bp

STRANDNESS: double

TOPOLOGY: linear

MOLECULE TYPE: genomic

ORIGINAL SOURCE ORGANISM: A. niger N400

IMMEDIATE EXPERIMENTAL SOURCE: pGW1800 of E. coli JM109/pGW1800 (DSM 5505)

FEATURES: from 1 to 203 bp PGII promoter region
from 204 to 261 bp signal peptide
from 262 to 818 bp N-terminal part of pro-PGII

PROPERTIES: 5' terminal part of the PGII gene of A. niger comprising promoter region, signal sequence and N-terminal of pro-PGII.

```
AACGAGGATC CAGGGTCCTA CATTTCCTCC AGGGGCTGTC               40
GGCAGTTATG AACTTTTCGA CCGGAAAAGA TTCGCAATAG               80
TCGTGAGTAT AAGAACCTCG TACCTGCTCA CACTGATGTC              120
TACTTGCTCA TCATTCCACA CTCATTCAAA ATCTTACCAA              160
CAACACTCCT TCTGTCATTC TTTTCTATTG TTAACAATTA ATC ATG      206
                                                MET


CAC TCG TTT GCT TCT CTT CTC GCC TAC GGC CTG GTC          242
His Ser Phe Ala Ser Leu Leu Ala Tyr Gly Leu Val
            -15                 -10

GCC GGC GCC ACC TTC GCT TCT GCC TCT CCT ATC GAA          278
Ala Gly Ala Thr Phe Ala Ser Ala Ser Pro Ile Glu
    -5                                  5

GCT CGA GAC AGC TGC ACG TTC ACC ACC GCT GCC GCT          314
Ala Arg Asp Ser Cys Thr Phe Thr Thr Ala Ala Ala
             10                  15
```

```
GCT AAA GCG GGC AAG GCG AAA TGC TCT ACT ATC ACC        350
Ala Lys Ala Gly Lys Ala Lys Cys Ser Thr Ile Thr
     20                      25                  30

CTT AAC AAC ATC GAA GTT CCA GCT GGA ACC ACC CTC        386
Leu Asn Asn Ile Glu Val Pro Ala Gly Thr Thr Leu
                 35                      40

GAC CTG ACC GGT CTC ACC AGC GGT ACC AAG GTC ATC        422
Asp Leu Thr Gly Leu Thr Ser Gly Thr Lys Val Ile
         45                      50

TTC GAG GGC ACC ACG ACC TTC CAG TAC GAA GAA TGG        458
Phe Glu Gly Thr Thr Thr Phe Gln Tyr Glu Glu Trp
 55                      60                      65

GCA GGC CCC TTG ATC TCC ATG AGT GGC GAA CAT ATC        494
Ala Gly Pro Leu Ile Ser Met Ser Gly Glu His Ile
                 70                      75

ACC GTC ACT GGT GCC TCC GGC CAC CTC ATC AAT TGC        530
Thr Val Thr Gly Ala Ser Gly His Leu Ile Asn Cys
     80                      85                  90

GAT GGT GCG CGC TGG TGG GAT GGC AAG GGA ACC AGC        566
Asp Gly Ala Arg Trp Trp Asp Gly Lys Gly Thr Ser
                 95                      100

GGA AAG AAG AAG CCC AAG TTC TTT TAC GCC CAT GGC        602
Gly Lys Lys Lys Pro Lys Phe Phe Tyr Ala His Gly
         105                     110

CTT GAC TCC TCG TCT ATT ACT GGA TTA AAC ATC AAA        638
Leu Asp Ser Ser Ser Ile Thr Gly Leu Asn Ile Lys
115                      120                     125
```

```
AAC ACC CCC CTT ATG GCG TTT AGT GTC CAG GCG AAT          674
Asn Thr Pro Leu Met Ala Phe Ser Val Gln Ala Asn
                130                 135

GAC ATT ACG TTT ACC GAT GTT ACC ATC AAT AAT GCG          710
Asp Ile Thr Phe Thr Asp Val Thr Ile Asn Asn Ala
    140                 145                 150

GAT GGC GAC ACC CAG GGT GGA CAC AAC ACT GAT GCG          746
Asp Gly Asp Thr Gln Gly Gly His Asn Thr Asp Ala
                155                 160

TTC GAT GTT GGC AAC TCG GTC GGG GTG AAT ATC ATT          782
Phe Asp Val Gly Asn Ser Val Gly Val Asn Ile Ile
        165                 170

AAG CCT TGG GTC CAT AAC CAG GAT GAC TGT CTT GCG GTT      821
Lys Pro Trp Val His Asn Gln Asp Asp Cys Leu Ala Val
175                 180                 185
```

<u>SEQ ID NO. 4</u>

SEQUENCE TYPE: Nucleotide with corresponding polypeptide
SEQUENCE LENGTH: 653 bp
STRANDNESS: double
TOPOLOGY: linear
MOLECULE TYPE: genomic
ORIGINAL SOURCE ORGANISM: <u>A. niger</u> N400
IMMEDIATE EXPERIMENTAL SOURCE: pGW1800 of <u>E. coli</u> JM109/pGW1800 (DSM 5505)
FEATURES: from 1 to 116 bp coding region for the C-terminal of pro-PGII
PROPERTIES: 3' terminal part of the PGII gene comprising the C-terminal of pro-PGII.

```
AG ATC TAT CTT CTT TGC GGG TCT GGT AGC TGC TCG GAC       38
   Ile Tyr Leu Leu Cys Gly Ser Gly Ser Cys Ser Asp
                    5                 10
```

```
TGG ACC TGG GAC GAT GTG AAA GTT ACC GGG GGG AAG           74
Trp Thr Trp Asp Asp Val Lys Val Thr Gly Gly Lys
         15                  20

AAG TCC ACC GCT TGC AAG AAC TTC CCT TCG GTG GCC          110
Lys Ser Thr Ala Cys Lys Asn Phe Pro Ser Val Ala
 25                 30                  35

TCT TGT TAG GCTGCTAGGT TGGTGAGTTG TAGCCCTAGC             149
Ser Cys END


TGAAATTCGT CTGCTTCGTC TGCTTCGTCT GCTTCGTCTG              189
CTTCGTCTGC TTCTTCTGCT TCGTCTGCTT TGTCTGCTTT              229
GTCTGCTTCG TCCACTTCGT CCACTTCGAC TGGTTAGATG              269
GGCCTTGTAA TAGTTTTTAG AGAGAACAGA ATATGTACAG              309
TAAGCCTTAG AGGTGGTACC GAGTTGTATA TTTATTTAAA              349
ATGTTACCTA TCGCGTGTCT TTATATTTAT AGCCTTTTAC              389
ATATATACGG AGCTACAGTG GATTATCTTA CAGCCCACAC              429
TCATCGTGCT GGGAACTACG TGAATGAATG CTCGGTTAGA              469
AGGCCTTGCT CACTGCCACA ACCAACCAGG AACCTTGGCA              509
GGTACATGCT TGGGCATTTT TGTCTGGCCC TATCTCTTTC              549
CAGATGGTGG TCTGGATGAG TCACGGCACG AGTAGATTGA              589
CCGCTACTCC AACCCGCGCA TAAAGCATAC GCCAGAAGTG              629
CAAGGGATAC AAGACAGCCA GCTG                               653
```

<u>SEQ ID NO. 5</u>

SEQUENCE TYPE: Nucleotide

SEQUENCE LENGTH: 20 bases

STRANDNESS: single

TOPOLOGY: linear

MOLECULE TYPE: synthetic

FEATURES: Identical with bases 1 to 20 of the coding strand of the PGII sequence with SEQ ID NO. 6

PROPERTIES: Oligonucleotide A for the construction of precise PGII fusions with a heterologous gene using PCR.

```
AACGAGGATC CAGGGTCCTA                                              20
```

SEQ ID NO. 6

SEQUENCE TYPE: Nucleotide

SEQUENCE LENGTH: 32 bases

STRANDNESS: single

TOPOLOGY: linear

MOLECULE TYPE: synthetic

FEATURES: from 1 to 12 complementary to the region of the IFN AM119 gene coding for amino acids 1 to 4 of mature IFN.
from 13 to 32 complementary to base positions 260 to 241 of the coding strand of the PGII sequence with SEQ ID No. 6

PROPERTIES: Oligonucleotide C useful for the construction of precise PGIIss-IFN AM119 fusion genes

```
AGGCAGATCA CAAGCGAAGG TGGCGCCGGC GA                                32
```

SEQ ID NO. 7

SEQUENCE TYPE: Nucleotide

SEQUENCE LENGTH: 272 bases

STRANDNESS: single, coding strand

TOPOLOGY: linear

MOLECULE TYPE: recombinant

IMMEDIATE EXPERIMENTAL SOURCE: PCR

FEATURES: from 1 to 203 A. niger PGII promoter region
from 204 to 258 A. niger PGII signal sequence
from 259 to 270 N-terminal of IFN BDBB hybrid (IFN AM119)

PROPERTIES: DNA2 comprises a perfect in frame fusion of the A. niger PGII signal sequence and the N-terminal amino acids 1 to 4 of mature IFN AM119 (BDBB hybrid). Useful for the construction of expression vectors for the production of IFN secreted from A. niger hosts.

```
AACGAGGATC CAGGGTCCTA CATTTCCTCC AGGGGCTGTC              40
GGCAGTTATG AACTTTTCGA CCGGAAAAGA TTCGCAATAG              80
TCGTGAGTAT AAGAACCTCG TACCTGCTCA CACTGATGTC             120
TACTTGCTCA TCATTCCACA CTCATTCAAA ATCTTACCAA             160
CAACACTCCT TCTGTCATTC TTTTCTATTG TTAACAATTA ATC ATG     206
                                                MET

CAC TCG TTT GCT TCT CTT CTC GCC TAC GGC CTG GTC         242
His Ser Phe Ala Ser Leu Leu Ala Tyr Gly Leu Val
            -15                 -10

GCC GGC GCC ACC TTC GCT TGT CAT CTG CCT                 272
Ala Gly Ala Thr Phe Ala Cys Asp Leu Pro
     -5
```

SEQ ID NO. 8

SEQUENCE TYPE: Nucleotide

SEQUENCE LENGTH: 32 bases

STRANDNESS: single

TOPOLOGY: linear

MOLECULE TYPE: synthetic

FEATURES: from 1 to 12 bp identical with bases 249-261 of the coding strand of the PGII gene with SEQ ID NO. 6.
from 13 to 32 bp identical with the coding region for the N-terminal amino acids 1 to 7 of the mature IFN AM119 (BDBB hybrid)

PROPERTIES: Oligonucleotide D for the construction of precise in frame fusions of the coding regions for the A. niger PGII signal sequence and IFN AM119.

```
GCCACCTTCG CTTGTGATCT GCCTCAGACT CA                      32
```

SEQ ID NO. 9

SEQUENC: TYPE: Nucleotide

SEQUENCE LENGTH: 20 bases
STRANDNESS: single
TOPOLOGY: linear
MOLECULE TYPE: synthetic
FEATURES: Complementary to a stretch in the 3' non-coding region of the coding strand of the IFN AM119 (BDBB hybrid) gene. Comprises an EcoRI site.
PROPERTIES: Oligonucleotide F for the construction of expression vectors for the expression of IFN AM119.

```
CCTGGGGGAA TTCAAAGTCA                                        20
```

SEQ ID NO. 10
SEQUENCE TYPE: linear
SEQUENCE LENGTH: 133 bases
STRANDNESS: single
TOPOLOGY: linear
MOLECULE TYPE: recombinant
FEATURES: from 1 to 12 C terminal of the A. niger PGII signal peptide
from 13 to 133 mature IFN AM119 (BDBB hybrid)
PROPERTIES: DNA3 comprises a perfect in frame fusion of the A. niger signal sequence C-terminal and the IFN AM119 (BDBB hybrid). Useful for the construction of expression vectors for the production of IFN secreted from A. niger hosts.

```
GCC ACC TTC GCT TGT GAT CTG CCT CAG ACT CAC AGC          36
Ala Thr Phe Ala Cys Asp Leu Pro Gln Thr His Ser


CTG GGT AAC AGG AGG GCC TTG ATA CTC CTG GCA CAA          72
Leu Gly Asn Arg Arg Ala Leu Ile Leu Leu Ala Gln

ATG CGA AGA ATC TCT CCT TTC TCC TGC CTG AAG GAC         108
Met Arg Arg Ile Ser Pro Phe Ser Cys Leu Lys Asp

AGA CAT GAC TTT GAA TTC CCC CAG G                       133
Arg His Asp Phe Glu Phe Pro Gln
```

SEQ ID NO. 11

SEQUENCE TYPE: Nucleotide

SEQUENCE LENGTH: 990 bp

STRANDEDNESS: double

TOPOLOGY: linear

MOLECULE TYPE: recombinant

IMMEDIATE EXPERIMENTAL SOURCE: Plasmid PGIIssIFN AM119

FEATURES: from 1 to 178 bp A. niger PGII promoter
from 199 to 255 bp A. niger PGII signal sequence
from 256 to 753 bp mature IFN AM119 (BDBB hybrid)
from 756 to 984 bp yeast PHO5 terminatór
from 1 to 6 bp BamHI site
from 364 to 369 bp EcoRI site
from 885 to 990 bp PstI site

PROPERTIES: BamHI-PstI-Fragment of pPGIIssIFN AM119 comprising the BamHI-EcoRI site generated by PCR which contains the perfect in frame fusion of the PGII signal sequence and the Interferon AM119 (BDBB hybrid) structural gene linked to the PGII promoter and PHO5 terminator.

```
          GG ATCCAGGGTC CTACATTTCC TCCAGGGGCT GTCGGCAGTT 42

ATGAACTTTT CGACCGGAAA AGATTCGCAA TAGTCGTGAG TATAAGAACC 92

TCGTACCTGC TCACACTGAT GTCTACTTGC TCATCATTCC ACACTCATTC 142

AAAATCTTAC CAACAACACT CCTTCTGTCA TTCTTTTCTA TTGTTAACAA 192

TTAATCATGC ACTCGTTTGC TTCTCTTCTC GCCTACGGCC TGGTCGCCGG 242

CGCCACCTTC GCTTGTGATC TGCCTCAGAC TCACAGCCTG GGTAACAGGA 292

GGGCCTTGAT ACTCCTGGCA CAAATGCGAA GAATCTCTCC TTTCTCCTGC 342
```

```
CTGAAGGACA GACATGACTT TGAATTCCCC CAGGAGGAGT TTGATGATAA 392
ACAGTTCCAG AAGGCTCAAG CCATCTCTGT CCTCCATGAG ATGATCCAGC 442
AGATCTTCAA CCTCTTTACC ACAAAAGATT CATCTGCTGC TTGGGATGAG 492
GACCTCCTAG ACAAATTCTG CACCGAACTC TACCAGCAGC TGAATGACCT 542
GGAGTCCTGT GTGATGCAGG AAGTGGGGGT GATAGAGTCT CCCCTGATGT 592
ACGAGGACTC CATCCTGGCT GTGAGGAAAT ACTTCCAAAG AATCACTCTA 642
TATCTGACAG AGAAGAAATA CAGCTCTTGT GCCTGGGAGG TTGTCAGAGC 692
AGAAATCATG AGATCCTTCT CTTTATCAAT CAACTTGCAA AAAAGATTGA 742
AGAGTAAGGA ATGAGACCTG GTACAACACG GAAATGATTC TTATAGACTA 792
ATACAGCAGC TCACACTTCG TCGAGGGTCA GCAGCGTCAG TAACTCTACT 842
GAATTGACCT TCTACTGGGA CTGGAACACT ACTCATTACA ACGCCAGTCT 892
ATTGAGACAA TAGTTTTGTA TAACTAAATA ATATTGGAAA CTAAATACGA 942
ATACCCAAAT TTTTTATCTA AATTTTGCCG AAAGATTAAA ATCTGCAG   990
```

SEQ ID NO. 12

SEQUENCE TYPE: Nucleotide

SEQUENCE LENGTH: 45 bases

STRANDNESS: single

TOPOLOGY: linear

MOLECULE TYPE: synthetic

FEATURES: from base 1 to 16 identical with bases 1026 to 1041 of the pki promoter

region (SEQ ID NO. 1)
from base 17 to 19 startcodon ATG
from base 20 to 45 amino acids 1 to 9 of mature IFN AM 119 (BDBB hybrid)

PROPERTIES: Oligonucleotide IFN-1, useful for the preparation of perfect fusions of the A. niger pki promoter and the IFN AM119 structural gene using PCR

```
TCAATCATCC GTCAAGATGT GTGATCTGCC TCAGACTCAC        40
AGCCT                                              45
```

SEQ ID NO. 13
SEQUENCE TYPE: Nucleotide
SEQUENCE LENGTH: 42 bases
STRANDNESS: single
TOPOLOGY: linear
MOLECULE TYPE: synthetic
FEATURES: from base 1 to 21 identical with bases 1031 to 1041 of the coding strand of the pki gene (SEQ ID NO. 1, promoter region).
from base 22 to 42 identical with the bases 204 to 224 of the coding strand of the PGII gene (SEQ ID NO. 6, signal sequence).
PROPERTIES: Oligonucleotide IFN-2, useful for the preparation of perfect fusions of the A. niger pki promoter and the A. niger PGII signal sequence using PCR.

```
AGAGATCAAT CATCCGTCAA GATGCACTCG TTTGCTTCTC        40
TT                                                 42
```

SEQ ID NO. 14
SEQUENCE TYPE: Nucleotide
SEQUENCE LENGTH: 1901 bp
STRANDEDNESS: double

TOPOLOGY: linear

MOLECULE TYPE: recombinant

IMMEDIATE EXPERIMENTAL SOURCE: Plasmid pPKI-IFN-2

FEATURES: from 1 to 6 bp BamHI site
from 1 to 742 bp identical with the pki promoter fragment extending from position 300 up to 1041 of the sequence with SEQ ID NO. 1
from 742 to 744 bp startcodon ATG
from 745 to 1243 bp Structural gene for IFN AM119 (BDBB hybrid).
from 1244 to 1245 bp stopcodon TGA
from 1247 to 1476 bp yeast PHO5 terminator fragment
from 1477 to 1842 bp SphI site
from 1483 to 1894 bp A. niger pki terminator
from 1896 to 1901 bp EcoRI site

PROPERTIES: BamHI/EcoRI-fragment of pPKI-IFN-2. Comprises an A. niger pki promoter fragment fused to a DNA sequence comprising a start codon, the IFN AM119 structural gene and part of the yeast PHO5 terminator region as well as the A. niger pki terminator.

```
GGATCCGCGAC GATGTGGGGT CAACTGGACA GAGTGAGAGG GTATCATGTG    51

GTCCTGCCAG ATACTTCGCA GAATGTTGTG TGGGTGTCTG ATTGTGGCTT    101

GGGCGTGAAT TGCTTTTGGT CTTCCCAACC AATTATTATT GCATGCGGCG    151

TATGAATGCC TGAGATGCGC GGAGGGAAGG TGCCTGAGGA TGTAGTGGAC    201

AAATGCTGCT GATCGCTGGG CGGAAACCCT TGGCTGACCA GTGAAAAGAG    251

CGGACGGAGG CAGCAGGTGT ATCTACGATC AAAGAATAGT AGCAAAGCAG    301

TGAAAGGTGG ATCACCCAGC AAATAATTGA GTTTTGATAC CCAGCGATAG    351

TGCCGGGGGG GAGAAAAAGT CATTAATAAT GGGAATTATG TAGGCGATGG    401
```

```
GAAGTGTGAT TGTAACTACT CCGTAGCTGG AGGCACAACT AACAAGCCAG    451
CTCTCAACCC GCGGGGAACC GACCGACnGA TAAAAAAAAG CGTCCCAAAG    501
CAGGAATCCC ACCAAAAAGG GCCGATCCAG CCAATCACCG CCGCCAACAT    551
TTTTCCTTCC CGGGCACCCC TCCTCTAGTC CACCATCTCT CTCTTCTCTC    601
GCTCACCGGC CCCGTCTTTT CCTTCCCTAT TATCTCTCCC TCTTCTCCTC    651
CCTTCTCTCC CTCCATTCTT TCTCCCATCT TCATCACTCC CTTCTCTTCT    701
GTCTTCCCCC CCGGTTCAGT AGAGATCAAT CATCCGTCAA GATGTGTGAT    751
CTGCCTCAGA CTCACAGCCT GGGTAACAGG AGGGCCTTGA TACTCCTGGC    801
ACAAATGCGA AGAATCTCTC CTTTCTCCTG CCTGAAGGAC AGACATGACT    851
TTGAATTCCC CCAGGAGGAG TTTGATGATA AACAGTTCCA GAAGGCTCAA    901
GCCATCTCTG TCCTCCATGA GATGATCCAG CAGATCTTCA ACCTCTTTAC    951
CACAAAAGAT TCATCTGCTG CTTGGGATGA GGACCTCCTA GACAAATTCT   1001
GCACCGAACT CTACCAGCAG CTGAATGACC TGGAGTCCTG TGTGATGCAG   1051
GAAGTGGGGG TGATAGAGTC TCCCCTGATG TACGAGGACT CCATCCTGGC   1101
TGTGAGGAAA TACTTCCAAA GAATCACTCT ATATCTGACA GAGAAGAAAT   1151
ACAGCTCTTG TGCCTGGGAG GTTGTCAGAG CAGAAATCAT GAGATCCTTC   1201
TCTTTATCAA TCAACTTGCA AAAAAGATTG AAGAGTAAGG AATGAGACCT   1251
GGTACAACAC GGAAATGATT CTTATAGACT AATACAGCAG CTCACACTTC   1301
```

```
GTCGAGGGTC AGCAGCGTCA GTAACTCTAC TGAATTGACC TTCTACTGGG  1351
ACTGGAACAC TACTCATTAC AACGCCAGTC TATTGAGACA ATAGTTTTGT  1401
ATAACTAAAT AATATTGGAA ACTAAATACG AATACCCAAA TTTTTTATCT  1451
AAATTTTGCC GAAAGATTAA AATCGGCATG CCACCGGTTG GTGGATGACA  1501
CGGTAACGAC AGCGATTGGA TAGAAAGCGT CAAGGGTGTG TCTCTGGATA  1551
TCGTAGACCG GTCTCGCCGC CATGCGATGA CTCAGGTAGT CTCCCCGCGG  1601
GCAGGCTGCT TGTGTCTTCA CTGCGAGACT CGTTAGTCGG GGCAGGACGA  1651
GGAGCACCAG GAGTGCGCAC TTGCGTCTAC CACCACATTC ACTTTGAGCC  1701
CGAAACGCGC TGGGGGAAGA CACACACCTT GGGAACGTAA TGTGTATGTA  1751
TCTTGATTTG ACGTTAGTTA GCCAGCCATG TTTGGTGGAC TTTGCTGCGA  1801
TCAAAAGCTA GAATTTAATG AGTTGTTCAC AATGCCTCGA TGAGATAAAG  1851
CACAGCATCC TGAGTGTCAG TCAGTATGAT TTGTTAGTGG AAAGGAATTC  1901
```

SEQ ID NO. 15

SEQUENCE TYPE: Nucleotide

SEQUENCE LENGTH: 2020 bp

STRANDEDNESS: double

TOPOLOGY: linear

MOLECULE TYPE: recombinant

IMMEDIATE EXPERIMENTAL SOURCE: Plasmid pPKIssIFN-2

FEATURES: from 1 to 6 bp BamHI site

from 1 to 742 bp identical with the pki promoter fragment extending from position 300 up to 1041 of the sequence with SEQ ID NO. 1

from 743 to 799 bp A. niger PGII signal sequence

from 800 to 1297 bp mature IFN AM119 (BDBB hybrid) structural gene
from 1298 to 1300 bp Stopcodon TGA
from 1301 to 1528 bp yeast PHO5 terminator fragment
from 1519 to 1534 bp SphI site
from 1535 to 1948 bp A. niger pki terminator
from 1950 to 1955 bp EcoRI site

PROPERTIES: BamHI/EcoRI-fragment of pPKIssIFN-2. Comprises an A. niger pki promoter fragment fused to the perfect in frame fusion of the PGII signal sequence and the IFN AM119 (BDBB hybrid) structural gene and to part of the yeast PHO5 terminator region as well as the A. niger pki terminator.

```
GGATCCGCGAC GATGTGGGGT CAACTGGACA GAGTGAGAGG GTATCATGTG  51

GTCCTGCCAG ATACTTCGCA GAATGTTGTG TGGGTGTCTG ATTGTGGCTT  101

GGGCGTGAAT TGCTTTTGGT CTTCCCAACC AATTATTATT GCATGCGGCG  151

TATGAATGCC TGAGATGCGC GGAGGGAAGG TGCCTGAGGA TGTAGTGGAC  201

AAATGCTGCT GATCGCTGGG CGGAAACCCT TGGCTGACCA GTGAAAAGAG  251

CGGACGGAGG CAGCAGGTGT ATCTACGATC AAAGAATAGT AGCAAAGCAG  301

TGAAAGGTGG ATCACCCAGC AAATAATTGA GTTTTGATAC CCAGCGATAG  351

TGCCGGGGGG GAGAAAAAGT CATTAATAAT GGGAATTATG TAGGCGATGG  401

GAAGTGTGAT TGTAACTACT CCGTAGCTGG AGGCACAACT AACAAGCCAG  451

CTCTCAACCC GCGGGGAACC GACCGACNGA TAAAAAAAAG CGTCCCAAAG  501

CAGGAATCCC ACCAAAAAGG GCCGATCCAG CCAATCACCG CCGCCAACAT  551
```

TTTTCCTTCC CGGGCACCCC TCCTCTAGTC CACCATCTCT CTCTTCTCTC 601

GCTCACCGGC CCCGTCTTTT CCTTCCCTAT TATCTCTCCC TCTTCTCCTC 651

CCTTCTCTCC CTCCATTCTT TCTCCCATCT TCATCACTCC CTTCTCTTCT 701

GTCTTCCCCC CCGGTTCAGT AGAGATCAAT CATCCGTCAA GATGCACTCG 751

TTTGCTTCTC TTCTCGCCTA CGGCCTGGTC GCCGGCGCCA CCTTCGCTTG 801

TGATCTGCCT CAGACTCACA GCCTGGGTAA CAGGAGGGCC TTGATACTCC 851

TGGCACAAAT GCGAAGAATC TCTCCTTTCT CCTGCCTGAA GGACAGACAT 901

GACTTTGAAT TCCCCCAGGA GGAGTTTGAT GATAAACAGT TCCAGAAGGC 951

TCAAGCCATC TCTGTCCTCC ATGAGATGAT CCAGCAGATC TTCAACCTCT 1001

TTACCACAAA AGATTCATCT GCTGCTTGGG ATGAGGACCT CCTAGACAAA 1051

TTCTGCACCG AACTCTACCA GCAGCTGAAT GACCTGGAGT CCTGTGTGAT 1101

GCAGGAAGTG GGGGTGATAG AGTCTCCCCT GATGTACGAG GACTCCATCC 1151

TGGCTGTGAG GAAATACTTC CAAAGAATCA CTCTATATCT GACAGAGAAG 1201

AAATACAGCT CTTGTGCCTG GGAGGTTGTC AGAGCAGAAA TCATGAGATC 1251

CTTCTCTTTA TCAATCAACT TGCAAAAAAG ATTGAAGAGT AAGGAATGAG 1301

ACCTGGTACA ACACGGAAAT GATTCTTATA GACTAATACA GCAGCTCACA 1351

CTTCGTCGAG GGTCAGCAGC GTCAGTAACT CTACTGAATT GACCTTCTAC 1401

TGGGACTGGA ACACTACTCA TTACAACGCC AGTCTATTGA GACAATAGTT 1451

```
TTGTATAACT AAATAATATT GGAAACTAAA TACGAATACC CAAATTTTTT 1501
ATCTAAATTT TGCCGAAAGA TTAAAATCGG CATGCCACCG GTTGGTGGAT 1551
GACACGGTAA CGACAGCGAT TGGATAGAAA GCGTCAAGGG TGTGTCTCTG 1601
GATATCGTAG ACCGGTCTCG CCGCCATGCG ATGACTCAGG TAGTCTCCCC 1651
GCGGGCAGGC TGCTTGTGTC TTCACTGCGA GACTCGTTAG TCGGGGCAGG 1701
ACGAGGAGCA CCAGGAGTGC GCACTTGCGT CTACCACCAC ATTCACTTTG 1751
AGCCCGAAAC GCGCTGGGGG AAGACACACA CCTTGGGAAC GTAATGTGTA 1801
TGTATCTTGA TTTGACGTTA GTTAGCCAGC CATGTTTGGT GGACTTTGCT 1851
GCGATCAAAA GCTAGAATTT AATGAGTTGT TCACAATGCC TCGATGAGAT 1901
AAAGCACAGC ATCCTGAGTG TCAGTCAGTA TGATTTGTTA GTGGAAAGGA 1951
ATTC                                                   1955
```

## Claims

**1.** A process for the preparation of a polypeptide characterized in that a structural gene coding for such polypeptide, except the homologous pki structural gene, is operatively linked with the A. niger pyruvate kinase transcription (pki) promoter extending from a nucleotide in about position 300 up to a nucleotide in about position 1042 of the DNA sequence described in SEQ ID NO 1 and is expressed in a suitable host.

**2.** A process according to claim 1 in which the host is filamentous fungus or a yeast.

**3.** A process according to claim 1 in which the host is not capable of expressing polygalacturonase or pectin lyase or which expresses polygalacturonase or pectin lyase in low amount.

**4.** A process according to claim 1 in which the host is selected from the group consisting of *A. niger*, *Neurospora crassa*, *Saccharomyces cerevisiae* and *Kluyveromyces lactis*.

**5.** A process according to claim 1 characterized in that human hybrid interferon BDBB is produced.

**6.** A process according to claim 1 characterized in that an A. niger pectin lyase is produced.

**7.** A process according to claim 1 characterized in that an A. niger polygalacturonase is produced.

## Patentansprüche

1. Verfahren zur Herstellung eines Polypeptids, dadurch gekennzeichnet, daß ein für ein derartiges Polypeptid codierendes Strukturgen, ausgenommen das homologe pki-Strukturgen, mit dem Promotor (pki) für die A. niger-Pyruvatkinase-Transkription, der sich von einem Nukleotid in der Position von etwa 300 bis zu einem Nukleotid in der Position von etwa 1042 der in SEQ ID NR. 1 beschriebenen DNA-Sequenz erstreckt, in funktionsfähigem Zustand gebunden ist und in einem geeigneten Wirt exprimiert wird.

2. Verfahren nach Anspruch 1, wobei der Wirt ein Fadenpilz oder eine Hefe ist.

3. Verfahren nach Anspruch 1, wobei der Wirt nicht in der Lage ist, Polygalacturonase oder Pectinlyase zu exprimieren oder welcher Polygalacturonase oder Pectinlyase in geringer Menge exprimiert.

4. Verfahren nach Anspruch 1, wobei der Wirt ausgewählt ist aus der Gruppe, bestehend aus A. niger, Neurospora crassa, Saccharomyces cerevisiae und Kluyveromyces lactis.

5. Verfahren nach Anspruch 1, dadurch gekennzeichnet, daß Humanhybridinterferon BDBB erzeugt wird.

6. Verfahren nach Anspruch 1, dadurch gekennzeichnet, daß eine A. niger-Pectinlyase erzeugt wird.

7. Verfahren nach Anspruch 1, dadurch gekennzeichnet, daß eine A. niger-Polygalacturonase erzeugt wird.

## Revendications

1. Un procédé pour la préparation d' un polypeptide, caractérisé en ce qu'un gène structurel qui est codant pour un tel polypeptide, à l'exception du gène structurel homologue pki, est lié, de façon opérante, avec le promoteur de transcription de pyruvate kinase (pki) de A. niger, qui s'étend d'un nucléotide en position approximative 300 jusqu'à un nucléotide en position approximative 1042 d'une séquence d'ADN décrite dans SEQ ID NO.1 et est exprimé dans un hôte approprié.

2. Un procédé selon la revendication 1, dans lequel l'hôte est un mycète filamenteux ou une levure.

3. Un procédé selon la revendication 1, dans lequel l'hôte n'est pas capable d'exprimer une polygalacturonase ni une lyase de pectine ou bien qui exprime une polygalacturonase ou bien une pectine lyase en faible quantité.

4. Un procédé selon la revendication 1, dans lequel l'hôte est choisi dans le groupe constitué par A. Niger, Neurospora crassa, Saccharomyces cerevisiae et Kluyveromyces lactis.

5. Un procédé selon la revendication 1, caractérisé en ce que l'interféron hybride humain BDBB est préparé.

6. Un procédé selon la revendication 1, caractérisé en ce qu'une lyase de pectine A. Niger est préparée.

7. Un procédé selon la revendication A, caractérisé en ce qu'une polygalacturonase de A. Niger est préparée.